# EUROPEAN PATENT APPLICATION

(11) **EP 4 549 555 A2**
(43) Date of publication of application: **07.05.2025**
(21) Application number: 25152170.4
(22) Date of filing: 22.05.2020
(51) Int. Cl.: C12N 5/0783

(54) **CCR4 TARGETED CHIMERIC ANTIGEN RECEPTOR MODIFIED T CELLS FOR TREATMENT OF CCR4 POSITIVE MALIGNANCIES**

(30) Priority: 24.05.2019 US 201962852934 P
(62) Divisional of application: 20733103.4
(71) Applicant: City of Hope, Duarte, CA 91010 (US)
(72) Inventor: BUDDE, Lihua Elizabeth, Duarte, 91010 (US); DEL REAL, Marissa M., Duarte, 91010 (US); FORMAN, Stephen, Duarte, 91010 (US)
(74) Representative: Fish & Richardson P.C.

(57) **Abstract**

Chimeric antigen receptors for use in treating lymphoma-associated C-C chemokine receptor type 4 (CCR4) and other cancers expressing CCR4 are described.

## Description

### CLAIM OF PRIORITY

This application claims the benefit of U.S. Provisional Application Serial No. 62/852,934, filed on May 24, 2019. The entire contents of the foregoing are incorporated herein by reference.

### TECHNICAL FIELD

This disclosure concerns lymphoma-associated C-C chemokine receptor type 4 (CCR4)-specific chimeric antigen receptor (CAR)-engineered T cells, methods of formulating, and methods of use as anti-cancer agents selective against CCR4-positive cells.

### BACKGROUND

CCR4 is expressed at high levels on the malignant skin-homing T cells, and its surface expression is closely associated with the enhanced skin-homing characteristics of cutaneous T cell lymphoma (CTCL) cells and unfavorable disease outcomes (Chang D-H, Sui J, Geng S, Muvaffak A, Bai M, Fuhlbrigge RC, et al. Humanization of an anti-CCR4 antibody that kills Cutaneous T-Cell Lymphoma cells and abrogates suppression by T-regulatory cells. Mol. Cancer Ther. 2012; 11:2451-61). A CAR that targets CCR4 using humanized variable heavy and (Vh) and kappa light (Vl) chain moieties can be derived from an anti-CCR4 antibody that is different from mogamulizumab (Perera LP, Zhang M, Nakagawa M, et al. Chimeric antigen receptor modified T cells that target chemokine receptor CCR4 as a therapeutic modality for T cell malignancies. Am. J. Hematol. 2017; 92(9):892-901).

### SUMMARY

Described herein are methods for using CCR4 targeted CAR T cells (also herein called CCR4 CAR T cells) to treat a variety of cancers, for example, cutaneous T cell lymphoma (CTCL). In addition methods of use as anti-cancer agents selective against CCR4-positive cells, also described herein are methods of decreasing the population of regulatory T cells (Tregs). Without being bound by theory, Tregs can interfere with the desired immune response and methods described here can be used to eliminate or reduce the number of CCR4-positive Tregs.

Described herein is a nucleic acid molecule comprising a nucleotide sequence encoding a chimeric antigen receptor (CAR) or polypeptide, wherein the chimeric antigen receptor or polypeptide comprises: an scFv targeting CCR4, a spacer, a transmembrane domain, a 41-BB co-stimulatory domain, and a CD3 ζ signaling domain.

In various embodiments: the transmembrane domain is selected from: a CD4 transmembrane domain or variant thereof having 1-5 amino acid modifications, a CD8 transmembrane domain or variant thereof having 1-5 amino acid modifications, a CD28 transmembrane domain or a variant thereof having 1-5 amino acid modifications; the spacer comprises 20-150 amino acids and is located between the scFv and the transmembrane domain; the transmembrane domain is a CD4 transmembrane domain or variant thereof having 1-5 amino acid modifications; the transmembrane domain is a CD4 transmembrane domain; the chimeric antigen receptor comprises a transmembrane domain selected from: a CD4 transmembrane domain or variant thereof having 1-2 amino acid modifications, a CD8 transmembrane domain or variant thereof having 1-2 amino acid modifications, a CD28 transmembrane domain or a variant thereof having 1-2 amino acid modifications; the spacer region comprises an amino acid sequence selected from the group consisting of SEQ ID NOs: 2-12 or a variant thereof having 1-5 amino acid modifications; the spacer comprises an IgG hinge region; the spacer comprises 10-50 amino acids; the 4-1BB costimulatory domain comprises the amino acid sequence of SEQ ID NO: 24 or a variant thereof having 1-5 amino acid modifications; the CD3ζ signaling domain comprises the amino acid sequence of SEQ ID NO:21; a linker of 3 to 15 amino acids is located between the 4-1BB costimulatory domain and the CD3 ζ signaling domain or variant thereof; the CAR or polypeptide comprises the amino acid sequence of SEQ ID NO: 29 or a variant thereof having 1-5 amino acid modifications; the scFv comprises the amino acid sequence of SEQ ID NO:1; the nucleic acid molecule of claim 1.

Also disclosed herein is: a viral vector comprising a nucleic acid molecule described herein; a population of human T cells (e.g., a population comprising central memory T cells) transduced by a vector comprising a nucleic acid molecule described herein. In some embodiments, the vector is an expression vector in which expression of the CCR4 CAR or CCR4 polypeptide is under the control of an inducible promoter. In some embodiments, the expression of the CCR4 CAR or CCR4 polypeptide is under the control of a Tet Off system.

Also described herein is a method of treating CCR4 positive cancers (including, e.g., peripheral T cell lymphoma, adult T cell lymphoma, anaplastic large cell lymphoma, primary cutaneous T cell lymphoma, renal cell carcinoma, lung cancer, hepatocellular carcinoma, and diffuse large B-cell lymphoma) in a patient comprising administering a population of autologous or allogeneic human T cells transduced by a vector comprising a nucleic acid molecule described herein, wherein the T cell lymphoma comprises cells expressing CCR4. In various embodiments: the chimeric antigen receptor or polypeptide is administered locally or systemically; the CCR4-expressing cells are cancerous T cells; and the chimeric antigen receptor or polypeptide is administered by single or repeat dosing.

In various embodiments: the chimeric antigen receptor or polypeptide comprises: a huCCR4 scFv (e.g., an scFv comprising the amino acid sequence QVQLVQSGAEVVKPGASVKISCKASGYTFTDHAIHWVKQNPGQRLEWIGYFSPGND DFKYNERFKGKATLTADTSASTAYVELSSLRSEDTAVYFCTRSLNMAYWGQGTLVT VSSGSTSGGGSGGGSGGGGSSDIVMSQSPDSLAVSLGERVTLNCKSSQSLLYSGNQK NYLAWYQQKPGQSPKLLIYWASARESGVPDRFSGSGSGTDFTLTISSVQAEDVAVYY CQQYYSYPLTFGAGTKLELK (SEQ ID NO:1) with up to 10 single amino acid substitutions).

In various embodiments: the chimeric antigen receptor or polypeptide comprises: a huCCR4 scFv (e.g., an scFv comprising the amino acid sequence QVQLVQSGAEVKKPGASVKVSCKASGYTFASYYMHWMRQAPGQGLEWIGWINPG NVNTKYNEKFKGRATLTVDTSTNTAYMELSSLRSEDTAVYYCARSTYYRPLDYWG QGTLVTVSSSGGGSGGGGSGGGGSDIVMTQSPDSLAVSLGERATINCKSSQSILYSSN QKNYLAWYQQKPGQSPKLLIYWASTRESGVPDRFSGSGSGTDFTLTISSLQAEDVAV YYCHQYLSSYTFGQGTKLEIK (SEQ ID NO:43) with up to 10 single amino acid substitutions).

In various embodiments: the chimeric antigen receptor or polypeptide comprises: a huCCR4 scFv (e.g., an scFv comprising the amino acid sequence QVQLVQSGAEVKKPGASVKVSCKASGYTFASYYMHWMRQAPGQGLEWIGWINPG NVNTKYNEKFKGRATLTVDTSTNTAYMELSSLRSEDTAVYYCARSTYYRPLDYWG QGTLVTVSSSGGGGSDIVMTQSPDSLAVSLGERATINCKSSQSILYSSNQKNYLAWY QQKPGQSPKLLIYWASTRESGVPDRFSGSGSGTDFTLTISSLQAEDVAVYYCHQYLSS YTFGQGTKLEIK (SEQ ID NO:44) with up to 10 single amino acid substitutions).

In various embodiments: the chimeric antigen receptor or polypeptide comprises: a huCCR4 scFv (e.g., an scFv comprising the amino acid sequence QVQLVQSGAEVKKPGASVKVSCKASGYTFASYYMHWMRQAPGQGLEWIGWINPG NVNTKYNEKFKGRATLTVDTSTNTAYMELSSLRSEDTAVYYCARSTYYRPLDYWG QGTLVTVSSGGGGSGGGGSDIVMTQSPDSLAVSLGERATINCKSSQSILYSSNQKNYL AWYQQKPGQSPKLLIYWASTRESGVPDRFSGSGSGTDFTLTISSLQAEDVAVYYCHQ YLSSYTFGQGTKLEIK (SEQ ID NO:45) with up to 10 single amino acid substitutions).

Also described a T cells harboring a vector expressing the CAR or polypeptide. In various embodiments: at least 20%, 30%, or 40% of the transduced human T cells are central memory T cells; at least 30% of the transduced human T cells are CD4+ and CD62L+ or CD8+ and CD62L+; the population of human T cells are autologous to the patient; and the population of human T cells are allogenic to the patient.

### CCR4 Targeted CAR

The CCR4 targeted CAR or CCR4 targeted polypeptide described herein include a CCR4 targeting scFv. In some embodiments, an scFv comprising the amino acid sequence: QVQLVQSGAEVKKPGASVKVSCKASGYTFASYYMHWMRQAPGQGLEWIGWINPG NVNTKYNEKFKGRATLTVDTSTNTAYMELSSLRSEDTAVYYCARSTYYRPLDYWG QGTLVTVSSSGGGSGGGGSGGGGSDIVMTQSPDSLAVSLGERATINCKSSQSILYSSN QKNYLAWYQQKPGQSPKLLIYWASTRESGVPDRFSGSGSGTDFTLTISSLQAEDVAV YYCHQYLSSYTFGQGTKLEIK (SEQ ID NO:43) or comprising the sequence QVQLVQSGAEVKKPGASVKVSCKASGYTFASYYMHWMRQAPGQGLEWIGWINPG NVNTKYNEKFKGRATLTVDTSTNTAYMELSSLRSEDTAVYYCARSTYYRPLDYWG QGTLVTVSS (SEQ ID NO:32) and the sequence DIVMTQSPDSLAVSLGERATMSCKSSQSILYSSNQKNYLAWYQQKPGQSPKLLIYWA STRESGVPDRFSGSGSGTDFTLTISSLQAEDVAVYYCHQYLSSYTFGQGTKLEIK (SEQ ID NO:33) joined by a flexible linker.

In some embodiments, an scFv comprises the amino acid sequence: QVQLVQSGAEVKKPGASVKVSCKASGYTFASQWMHWMRQAPGQGLEWIGWINPG NVNTKYNEKFKGRATLTVDTSTNTAYMELSSLRSEDTAVYYCARSTWYRPLDYWG QGTLVTVSS (SEQ ID NO:34) and the sequence DIVMTQSPDSLAVSLGERATMSCKSSQSILYSSNQKNYLAWYQQKPGQSPKLLIYWA STRESGVPDRFSGSGSGTDFTLTISSLQAEDVAVYYCHQYISSYTFGQGTKLEIK (SEQ ID NO:35) joined by a flexible linker.

In some embodiments, an scFv comprises the amino acid sequence: QVQLVQSGAEVKKPGASVKVSCKASGYTFASAWMHWMRQAPGQGLEWIGWINPG NVNTKYNEKFKGRATLTVDTSTNTAYMELSSLRSEDTAVYYCARSTYYRPLDYWG QGTLVTVSS (SEQ ID NO:36) and the sequence DIVMTQSPDSLAVSLGERATMSCKSSQSILYSSNQKNYLAWYQQKPGQSPKLLIYWA STRESGVPDRFSGSGSGTDFTLTISSLQAEDVAVYYCHQYMSSYTFGQGTKLEIK (SEQ ID NO:37) joined by a flexible linker.

In some embodiments, a useful flexible linker is 1, 2, 3, 4, 5, 6, 7, 8, 9, or 10 repeats of the sequence GGGS (SEQ ID NO:46). In some embodiments, a useful flexible linker is 1, 2, 3, 4, 5, 6, 7, 8, 9, or 10 repeats of the sequence GGGGS (SEQ ID NO:47).

A useful CCR4 CAR or CCR4 polypeptide can consist of or comprises the amino acid sequence of SEQ ID NO:38, 39, or 40 (mature CAR lacking a signal sequence) or the CCR4 CAR or CCR4 polypeptide can consist of or comprise the amino acid sequence of SEQ ID NO:29, 30, or 31 (immature CAR having a GMCSFRa signal sequence). The CAR or polypeptide can be expressed in a form that includes a signal sequence, e.g., a human GM-CSF receptor alpha signal sequence (MLLLVTSLLLCELPHPAFLLIP; SEQ ID NO:43). The CAR or polypeptide can be expressed with additional sequences that are useful for monitoring expression, for example, a T2A skip sequence and a truncated EGFRt or truncated CD19. Thus, the CAR or polypeptide can comprise or consist of the amino acid sequence of SEQ ID Nos: 1, 29, 30, 31, 38, 39, 40, 43, 44, or 45 or can comprise or consist of an amino acid sequence that is at least 95%, 96%, 97%, 98% or 99% identical to SEQ ID Nos: 1, 29, 30, 31, 38, 39, 40, 43, 44, or 45. The CAR or polypeptide can comprise or consist of the amino acid sequence of any of SEQ ID Nos 1, 29, 30, 31, 38, 39, 40, 43, 44, or 45with up to 1, 2, 3, 4 or 5 amino acid changes (preferably conservative amino acid changes). The CAR or polypeptide can comprise SEQ ID NO:32 with up to 1, 2, 3, 4 or 5 amino acid changes (preferably conservative amino acid changes) and SEQ ID NO:33 with up to 1, 2, 3, 4 or 5 amino acid changes (preferably conservative amino acid changes) joined by a flexible linker. The CAR or polypeptide can comprise SEQ ID NO:34 with up to 1, 2, 3, 4 or 5 amino acid changes (preferably conservative amino acid changes) and SEQ ID NO:35 with up to 1, 2, 3, 4 or 5 amino acid changes (preferably conservative amino acid changes) joined by a flexible linker. The CAR or polypeptide can comprise SEQ ID NO:36 with up to 1, 2, 3, 4 or 5 amino acid changes (preferably conservative amino acid changes) and SEQ ID NO:37 with up to 1, 2, 3, 4 or 5 amino acid changes (preferably conservative amino acid changes) joined by a flexible linker.

In some embodiments, the nucleic acid encoding amino acid sequences SEQ ID NOs:1, 29-40, and 43-45 are codon optimized.

### Spacer Region

The CAR or polypeptide described herein can include a spacer located between the CCR4 targeting domain (i.e., a CCR4 targeted ScFv or variant thereof) and the transmembrane domain. A variety of different spacers can be used. Some of them include at least portion of a human Fc region, for example a hinge portion of a human Fc region or a CH3 domain or variants thereof. **Table 1** below provides various spacers that can be used in the CARs described herein.

**Table 1: Examples of Spacers**

| Name | Length | Sequence |
|---|---|---|
| a3 | 3 aa | AAA |
| linker | 10 aa | GGGSSGGGSG (SEQ ID NO:2) |
| IgG4 hinge (S→P) (S228P) | 12 aa | ESKYGPPCPPCP (SEQ ID NO:3) |
| IgG4 hinge | 12 aa | ESKYGPPCPSCP (SEQ ID NO:4) |
| IgG4 hinge (S228P)+ linker | 22 aa | ESKYGPPCPPCPGGGSSGGGSG (SEQ ID NO:5) |
| CD28 hinge | 39 aa | IEVMYPPPYLDNEKSNGTIIHVKGKHLCPSPLFPGPSKP (SEQ ID NO:6) |
| CD8 hinge-48aa | 48 aa | AKPTTTPAPRPPTPAPTIASQPLSLRPEACRPAAGGAVHTRGLDFACD (SEQ ID NO:7) |
| CD8 hinge-45aa | 45aa | TTTPAPRPPTPAPTIASQPLSLRPEACRPAAGGAVHTRGLDFACD (SEQ ID NO:8) |
| IgG4(HL-CH3) Also called IgG4(HL-ΔCH2) (includes S228P in hinge) | 129 aa | ESKYGPPCPPCPGGGSSGGGSGGQPREPQVYTLPPSQEEMTKNQVSLTCL VKGFYPSDIAVEWESNGQPENNYKTTPPVLDSDGSFFLYSRLTVDKSRWQE GNVFSCSVMHEALHNHYTQKSLSLSLGK (SEQ ID NO:9) |
| IgG4(L235E,N297Q) | 229 aa | ESKYGPPCPSCPAPEFEGGPSVFLFPPKPKDTLMISRTPEVTCVVVDVSQED PEVQFNWYVDGVEVHQAKTKPREEQFQSTYRVVSVLTVLHQDWLNGKEY KCKVSNKGLPSSIEKTISKAKGQPREPQVYTLPPSQEEMTKNQVSLTCLVKG FYPSDIAVEWESNGQPENNYKTTPPVLDSDGSFFLYSRLTVDKSRWQEGN VFSCSVMHEALHNHYTQKSLSLSLGK (SEQ ID NO:10) |
| IgG4(S228P, L235E,N297Q) | 229 aa | ESKYGPPCPPCPAPEFEGGPSVFLFPPKPKDTLMISRTPEVTCVVVDVSQED PEVQFNWYVDGVEVHQAKTKPREEQFQSTYRVVSVLTVLHQDWLNGKEY KCKVSNKGLPSSIEKTISKAKGQPREPQVYTLPPSQEEMTKNQVSLTCLVKG FYPSDIAVEWESNGQPENNYKTTPPVLDSDGSFFLYSRLTVDKSRWQEGN VFSCSVMHEALHNHYTQKSLSLSLGK (SEQ ID NO:11) |
| IgG4(CH3) Also called IgG4(ΔCH2) | 107 aa | GQPREPQVYTLPPSQEEMTKNQVSLTCLVKGFYPSDIAVEWESNGQPENN YKTTPPVLDSDGSFFLYSRLTVDKSRWQEGNVFSCSVMHEALHNHYTQKSL SLSLGK (SEQ ID NO:12) |

Some spacer regions include all or part of an immunoglobulin (e.g., IgG1, IgG2, IgG3, IgG4) hinge region, i.e., the sequence that falls between the CH1 and CH2 domains of an immunoglobulin, e.g., an IgG4 Fc hinge or a CD8 hinge. Some spacer regions include an immunoglobulin CH3 domain (called CH3 or ΔCH2) or both a CH3 domain and a CH2 domain. The immunoglobulin derived sequences can include one or more amino acid modifications, for example, 1, 2, 3, 4 or 5 substitutions, e.g., substitutions that reduce off-target binding.

The hinge/linker region can also comprise a IgG4 hinge region having the sequence ESKYGPPCPSCP (SEQ ID NO:4) or ESKYGPPCPPCP (SEQ ID NO:3). The hinge/linger region can also comprise the sequence ESKYGPPCPPCP (SEQ ID NO:3) followed by the linker sequence GGGSSGGGSG (SEQ ID NO:2) followed by IgG4 CH3 sequence GQPREPQVYTLPPSQEEMTKNQVSLTCLVKGFYPSDIAVEWESNGQPENNYKTTPPV LDSDGSFFLYSRLTVDKSRWQEGNVFSCSVMHEALHNHYTQKSLSLSLGK (SEQ ID NO:12). Thus, the entire linker/spacer region can comprise the sequence: ESKYGPPCPPCPGGGSSGGGSGGQPREPQVYTLPPSQEEMTKNQVSLTCLVKGFYPS DIAVEWESNGQPENNYKTTPPVLDSDGSFFLYSRLTVDKSRWQEGNVFSCSVMHEA LHNHYTQKSLSLSLGK (SEQ ID NO:11). In some cases, the spacer has 1, 2, 3, 4, or 5 single amino acid changes (e.g., conservative changes) compared to SEQ ID NO:11. In some cases, the IgG4 Fc hinge/linker region that is mutated at two positions (L235E; N297Q) in a manner that reduces binding by Fc receptors (FcRs).

### Transmembrane Domain

A variety of transmembrane domains can be used in the. **Table 2** includes examples of suitable transmembrane domains. Where a spacer region is present, the transmembrane domain (TM) is located carboxy terminal to the spacer region.

### Table 2: Examples of Transmembrane Domains

| **Name** | **Accession** | **Length** | **Sequence** |
|---|---|---|---|
| CD3z | J04132.1 | 21 aa | LCYLLDGILFIYGVILTALFL (SEQ ID NO:13) |
| CD28 | NM_006139 | 27aa | FWVLVVVGGVLACYSLLVTVAFIIFWV (SEQ ID NO:14) |
| CD28(M) | NM_006139 | 28aa | MFWVLVVVGGVLACYSLLVTVAFIIFWV (SEQ ID NO:15) |
| CD4 | M35160 | 22aa | MALIVLGGVAGLLLFIGLGIFF (SEQ ID NO:16) |
| CD8tm | NM_001768 | 21aa | IYIWAPLAGTCGVLLLSLVIT (SEQ ID NO:17) |
| CD8tm2 | NM_001768 | 23aa | IYIWAPLAGTCGVLLLSLVITLY (SEQ ID NO:18) |
| CD8tm3 | NM_001768 | 24aa | IYIWAPLAGTCGVLLLSLVITLYC (SEQ ID NO:19) |
| 41BB | NM_001561 | 27aa | IISFFLALTSTALLFLLFF LTLRFSVV (SEQ ID NO:20) |

### Costimulatory Domain

The costimulatory domain can be any domain that is suitable for use with a CD3ζ signaling domain. In some cases the co-signaling domain is a 4-1BB co-signaling domain that includes a sequence that is at least 90%, at least 95%, at least 98% identical to or identical to: KRGRKKLLYIFKQPFMRPVQTTQEEDGCSCRFPEEEEGGCEL (SEQ ID NO:24). In some cases, the 4-1BB co-signaling domain has 1, 2, 3, 4 of 5 amino acid changes (preferably conservative) compared to SEQ ID NO:24.

The costimulatory domain(s) are located between the transmembrane domain and the CD3ζ signaling domain. **Table 3** includes examples of suitable costimulatory domains together with the sequence of the CD3ζ signaling domain.

**Table 3: CD3ζ Domain and Examples of Costimulatory Domains**

| **Name** | **Accession** | **Length** | **Sequence** |
|---|---|---|---|
| CD3ζ | J04132.1 | 113 aa | RVKFSRSADAPAYQQGQNQLYNELNLGRREEYDVLDKRRGR DPEMGGKPRRKNPQEGLYNELQKDKMAEAYSEIGMKGERR RGKGHDGLYQGLSTATKDTYDALHMQALPPR (SEQ ID NO:21) |
| CD28 | NM_006139 | 42aa | RSKRSRLLHSDYMNMTPRRPGPTRKHYQPYAPPRDFAAYRS (SEQ ID NO: 22) |
| CD28gg* | NM_006139 | 42aa | RSKRSRGGHSDYMNMTPRRPGPTRKHYQPYAPPRDFAAYR S (SEQ ID NO:23) |
| 41BB | NM_001561 | 42 aa | KRGRKKLLYIFKQPFMRPVQTTQEEDGCSCRFPEEEEGGCEL (SEQ ID NO:24) |
| OX40 | | 42 aa | ALYLLRRDQRLPPDAHKPPGGGSFRTPIQEEQADAHSTLAKI (SEQ ID NO:25) |

In various embodiments: the costimulatory domain is selected from the group consisting of: a costimulatory domain depicted in **Table 3** or a variant thereof having 1-5 (e.g., 1 or 2) amino acid modifications, a CD28 costimulatory domain or a variant thereof having 1-5 (e.g., 1 or 2) amino acid modifications, a 4-1BB costimulatory domain or a variant thereof having 1-5 (e.g., 1 or 2) amino acid modifications and an OX40 costimulatory domain or a variant thereof having 1-5 (e.g., 1 or 2) amino acid modifications. In certain embodiments, a 4-1BB costimulatory domain or a variant thereof having 1-5 (e.g., 1 or 2) amino acid modifications in present. In some embodiments there are two costimulatory domains, for example a CD28 co-stimulatory domain or a variant thereof having 1-5 (e.g., 1 or 2) amino acid modifications (e.g., substitutions) and a 4-1BB co-stimulatory domain or a variant thereof having 1-5 (e.g., 1 or 2) amino acid modifications (e.g., substitutions). In various embodiments the 1-5 (e.g., 1 or 2) amino acid modification are substitutions. The costimulatory domain is amino terminal to the CD3ζ signaling domain and a short linker consisting of 2 - 10, e.g., 3 amino acids (e.g., GGG) is can be positioned between the costimulatory domain and the CD3ζ signaling domain.

### CD3ζ Signaling Domain

The CD3ζ Signaling domain can be any domain that is suitable for use with a CD3ζ signaling domain. In some cases, the CD3ζ signaling domain includes a sequence that is at least 90%, at least 95%, at least 98% identical to or identical to: RVKFSRSADAPAYQQGQNQLYNELNLGRREEYDVLDKRRGRDPEMGGKPRRKNPQ EGLYNELQKDKMAEAYSEIGMKGERRRGKGHDGLYQGLSTATKDTYDALHMQAL PPR (SEQ ID NO:21). In some cases, the CD3ζ signaling has 1, 2, 3, 4 of 5 amino acid changes (preferably conservative) compared to SEQ ID NO:21.

### Truncated EGFR or CD19

The CD3ζ signaling domain can be followed by a ribosomal skip sequence (e.g., LEGGGEGRGSLLTCGDVEENPGPR; SEQ ID NO:27) and a truncated EGFR having a sequence that is at least 90%, at least 95%, at least 98% identical to or identical to: LVTSLLLCELPHPAFLLIPRKVCNGIGIGEFKDSLSINATNIKHFKNCTSISGDLHILPVA FRGDSFTHTPPLDPQELDILKTVKEITGFLLIQAWPENRTDLHAFENLEIIRGRTKQHG QFSLAVVSLNITSLGLRSLKEISDGDVIISGNKNLCYANTINWKKLFGTSGQKTKIISN RGENSCKATGQVCHALCSPEGCWGPEPRDCVSCRNVSRGRECVDKCNLLEGEPREF VENSECIQCHPECLPQAMNITCTGRGPDNCIQCAHYIDGPHCVKTCPAGVMGENNTL VWKYADAGHVCHLCHPNCTYGCTGPGLEGCPTNGPKIPSIATGMVGALLLLLVVAL GIGLFM (SEQ ID NO:28). In some cases, the truncated EGFR has 1, 2, 3, 4 of 5 amino acid changes (preferably conservative) compared to SEQ ID NO:28. Alternatively the CD3ζ signaling domain can be followed by a ribosomal skip sequence (e.g., LEGGGEGRGSLLTCGDVEENPGPR; SEQ ID NO:27) and a truncated CD19R (also called CD19t) having a sequence that is at least 90%, at least 95%, at least 98% identical to or identical to:

MPPPRLLFFLLFLTPMEVRPEEPLVVKVEEGDNAVLQCLKGTSDGPTQQLTWSRESP
LKPFLKLSLGLPGLGIHMRPLAIWLFIFNVSQQMGGFYLCQPGPPSEKAWQPGWTVN
VEGSGELFRWNVSDLGGLGCGLKNRSSEGPSSPSGKLMSPKLYVWAKDRPEIWEGE
PPCVPPRDSLNQSLSQDLTMAPGSTLWLSCGVPPDSVSRGPLSWTHVHPKGPKSLLS
LELKDDRPARDMWVMETGLLLPRATAQDAGKYYCHRGNLTMSFHLEITARPVLWH
WLLRTGGWKVSAVTLAYLIFCLCSLVGILHLQRALVLRRKR (SEQ ID NO:26)

An amino acid modification refers to an amino acid substitution, insertion, and/or deletion in a protein or peptide sequence. An "amino acid substitution" or "substitution" refers to replacement of an amino acid at a particular position in a parent peptide or protein sequence with another amino acid. A substitution can be made to change an amino acid in the resulting protein in a non-conservative manner (i.e., by changing the codon from an amino acid belonging to a grouping of amino acids having a particular size or characteristic to an amino acid belonging to another grouping) or in a conservative manner (i.e., by changing the codon from an amino acid belonging to a grouping of amino acids having a particular size or characteristic to an amino acid belonging to the same grouping). Such a conservative change generally leads to less change in the structure and function of the resulting protein. The following are examples of various groupings of amino acids: 1) Amino acids with nonpolar R groups: Alanine, Valine, Leucine, Isoleucine, Proline, Phenylalanine, Tryptophan, Methionine; 2) Amino acids with uncharged polar R groups: Glycine, Serine, Threonine, Cysteine, Tyrosine, Asparagine, Glutamine; 3) Amino acids with charged polar R groups (negatively charged at pH 6.0): Aspartic acid, Glutamic acid; 4) Basic amino acids (positively charged at pH 6.0): Lysine, Arginine, Histidine (at pH 6.0). Another grouping may be those amino acids with phenyl groups: Phenylalanine, Tryptophan, and Tyrosine.

In some cases, the CCR4 CAR or CCR4 polypeptide can be produced using a vector in which the CAR open reading frame is followed by a T2A ribosome skip sequence and a truncated EGFR (EGFRt), which lacks the cytoplasmic signaling tail. In this arrangement, co-expression of EGFRt provides an inert, non-immunogenic surface marker that allows for accurate measurement of gene modified cells, and enables positive selection of gene-modified cells, as well as efficient cell tracking of the therapeutic T cells in vivo following adoptive transfer. Efficiently controlling proliferation to avoid cytokine storm and off-target toxicity is an important hurdle for the success of T cell immunotherapy. The EGFRt incorporated in the CCR4 CAR lentiviral vector can act as suicide gene to ablate the CAR+ T cells in cases of treatment-related toxicity.

The CAR or polypeptide described herein can be produced by any means known in the art, though preferably it is produced using recombinant DNA techniques. Nucleic acids encoding the several regions of the chimeric receptor can be prepared and assembled into a complete coding sequence by standard techniques of molecular cloning known in the art (genomic library screening, overlapping PCR, primer-assisted ligation, site-directed mutagenesis, etc.) as is convenient. The resulting coding region is preferably inserted into an expression vector and used to transform a suitable expression host cell line, preferably a T lymphocyte, and most preferably an autologous T lymphocyte.

Various T cell subsets isolated from the patient can be transduced with a vector for CAR or polypeptide expression. Central memory T cells are one useful T cell subset. Central memory T cell can be isolated from peripheral blood mononuclear cells (PBMC) by selecting for CD45RO+/CD62L+ cells, using, for example, the CliniMACS^{®} device to immunomagnetically select cells expressing the desired receptors. The cells enriched for central memory T cells can be activated with anti-CD3/CD28, transduced with, for example, a lentiviral vector that directs the expression of an CCR4 CAR as well as a non-immunogenic surface marker for in vivo detection, ablation, and potential ex vivo selection. The activated/genetically modified CCR4 central memory T cells can be expanded *in vitro* with IL-2/IL-15 and then cryopreserved. Additional methods of preparing CAR T cells can be found in PCT/US2016/043392.

Methods for preparing useful T cell populations are described in, for example, WO 2017/015490 and WO 2018/102761. In some cases, it may be useful to use natural killer (NK) cells, e.g., allogenic NK cells derived from peripheral blood or cord blood. In other cases, NK cells can be derived from human embryonic stem cells (hESCs) or induced pluripotent stem cells (iPSCs).

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. All publications, patent applications, patents, sequences, database entries, and other references mentioned herein are incorporated by reference in their entirety for any and all purposes. In case of conflict, the present specification, including definitions, will control.

Other features and advantages of the invention will be apparent from the following detailed description and figures, and from the claims.

### DESCRIPTION OF DRAWINGS

**FIG. 1** shows a schematic depicting three CCR4 CAR constructs differing in the spacer region.
**FIG. 2** shows a schematic of CCR4 CAR design: Three different CCR4 CAR constructs were designed. All three of the constructs express the same codon optimized, humanized CCR4 single chain variable fragment. All of the CAR constructs also express a CD4 transmembrane (TM) domain, a 41BB costimulatory domain, a CD3 zeta (ζ) domain, and a separate protein (truncated EGFR) to mark the successful transduction of the cells with the CAR construct. The three CCR4 CAR constructs differ in their spacer domains.

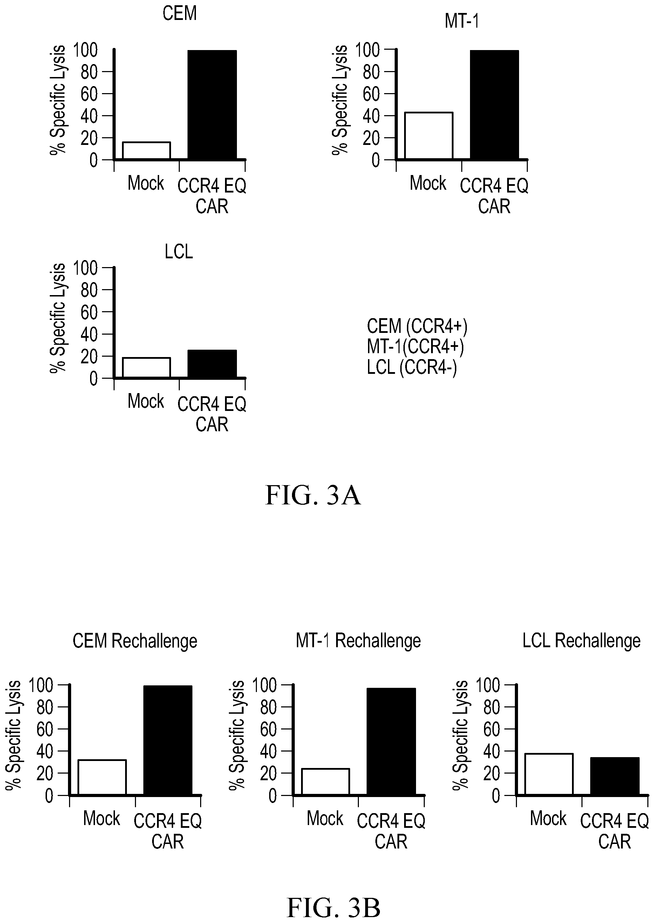

**FIGS. 3A-3B** shows results from a 48hr long term killing assay with CCR4 EQ CAR at 1:10 E:T **(A)** and rechallenge assays **(B).** In the rechallenge assays, after 2 days at an E:T of 1:2 tumor cells were added back so that the 1:2 E:T is re-established. Lysis was analyzed 48hrs later.
**FIG. 4** shows results from experiments with CCR4 CAR T cells where EGFRt is used as a tracking marker and its expression was stable through 7 day culture duration. Mock (untransduced) and CCR4 CAR T cells were evaluated by flow cytometry for EGFR expression to detect transduction of CARs.

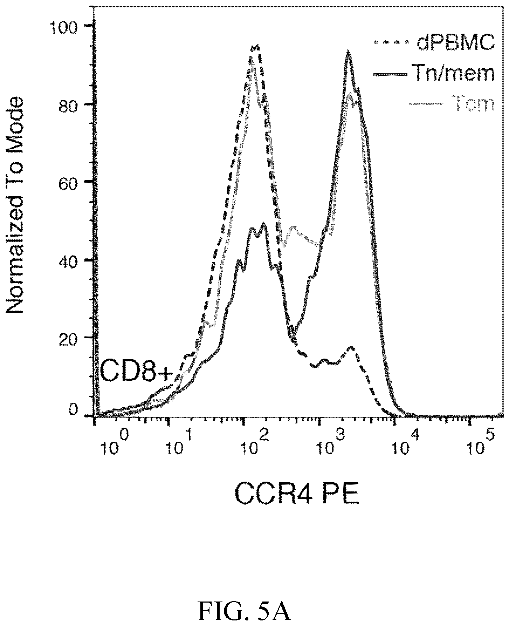

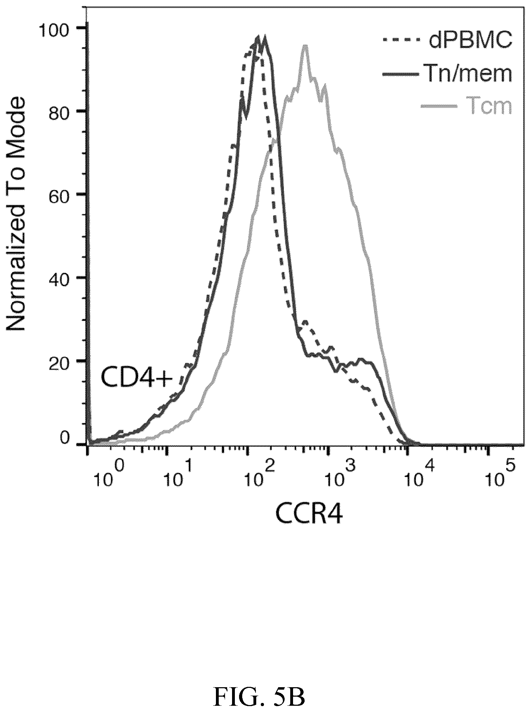

**FIGS. 5A-5B** show results of flow cytometric analysis of CCR4 expression in different T cell subpopulations. T cell populations of depleted PBMC (CD14-CD25-; also called "dPBCM"), naïve memory T cells (Tn/mem) and central memory T cells (Tcm) were stained for CCR4 expression via flow cytometry in CD8+ (FIG. 5A) and CD4+ (FIG. 5B).
**FIGS. 6A-6B** shows results from a CCR4 growth curve. (A) Pan T cells from the same healthy donor with the three different CCR4 CAR constructs and tracked the live cells counts of the cells over 13 days. (C) Depleted PBMC (CD14-CD25-; also called dPBCM) cells from the same healthy donor with the three different CCR4 CAR constructs and tracked the live cells counts of the cells over 13 days.
**FIGS. 7A-7D** shows the ability of the CCR4 CAR2 T cells to kill CCR4 expressing T cell tumor lines. (A) A schematic representation depicts a "Killing" assay followed by a "Rechallenge" assay demonstrates the ability of a CCR4 CAR T cell to kill CCR4 expressing T cell tumor lines. (B) Killing and rechallenge assays using CEM cells, a CCR4+ tumor cell line. (C) Killing and rechallenge assays using MT1 cells, a CCR4+ tumor cell line. (D) Killing and rechallenge assays using LCL cells, CCR4 negative B cell tumor line.
**FIGS. 8A-8C** shows survival curves in three mouse models engrafted with CCR4-expressing malignant T cell lines using a variety of injection routes (IP, intraperitoneal; SC subcutaneous; IV, intravenous).
**Figure 9A-9B** shows *in vivo* efficacy of the CCR4 CAR T cells. (A) A schematic depicts sc engraftment of CEM in NSG mice. After 4 days, mice were treated IV with the CAR T cells or mock transduced T cells and compared to a tumor only group. (B) Survival of mice treated IV with the CAR or mock transduced T cells compared to a tumor only group was monitored.

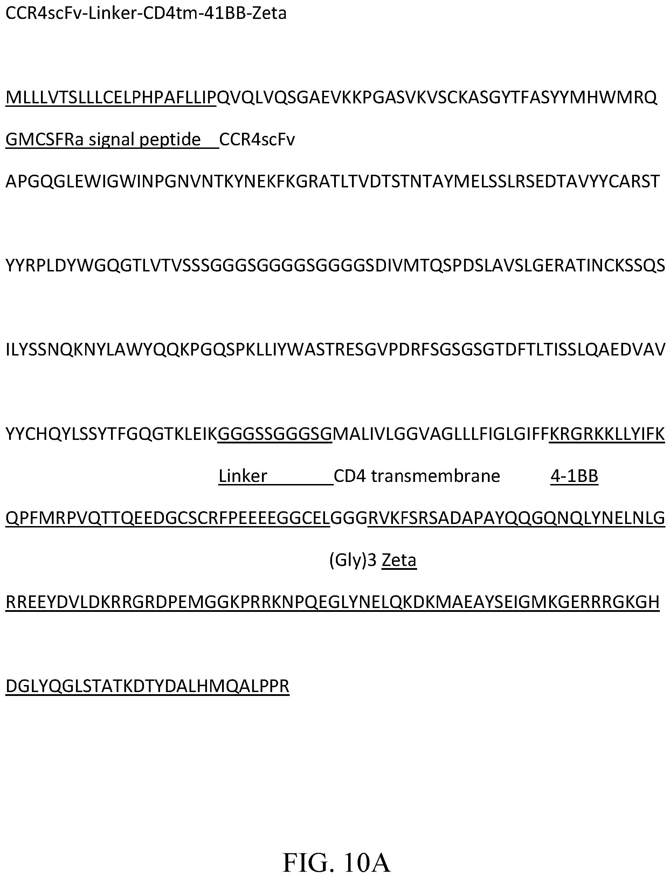

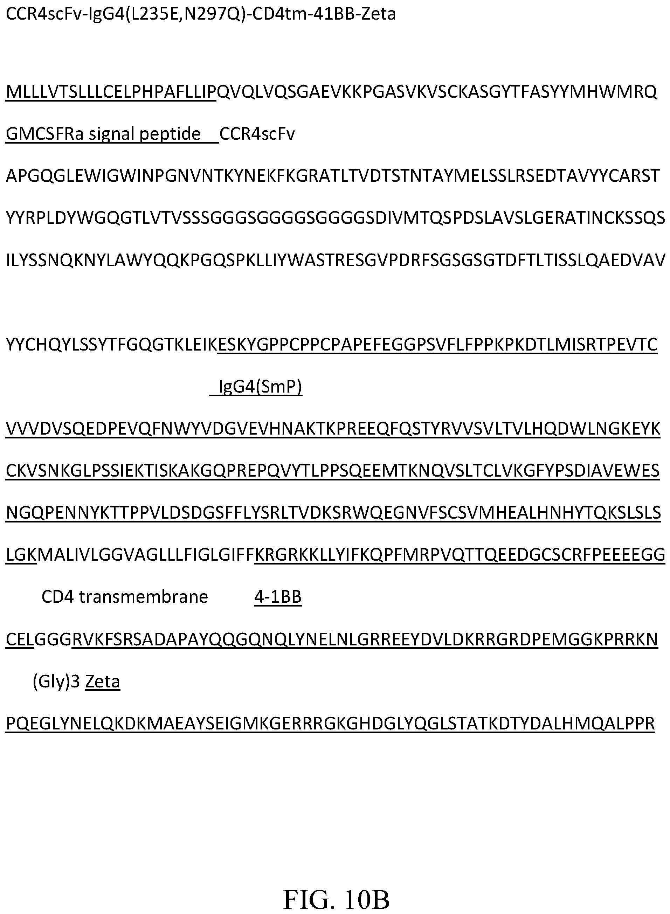

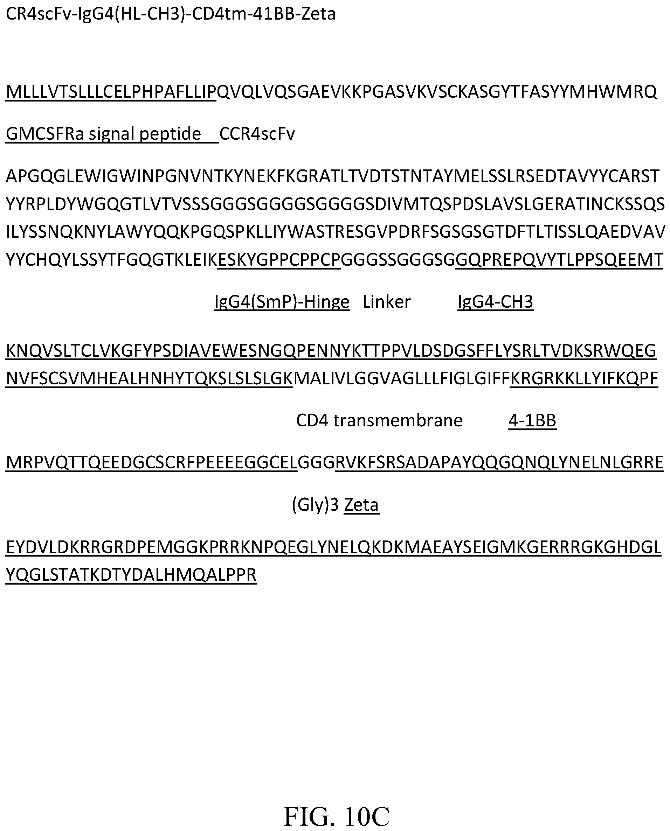

**Figure 10A-10C** show the annotated amino acid sequences of CAR1 (CCR4 L CAR) (A; SEQ ID NO: 29) CAR2 (CCR4 EQ CAR) (B; SEQ ID NO: 30), and CAR3 (CCR4 ΔCH2 CAR; also called CCR4 CH3 CAR) (C; SEQ ID NO: 31).
**FIG. 11** shows bioluminescent detection of tumor growth following engraftment of CEM in NSG mice treated IV with the CAR T cells or mock transduced T cells and compared to a tumor only group.
**FIGS. 12A-12B** show in vivo efficacy of the CCR4 CAR T cells in a HUT78 mouse model. (A) Bioluminescent detection of tumor growth following IV engraftment of HUT78 in NSG mice. After 10 days, mice were treated IV with the CAR T cells or mock transduced T cells. (B) Survival of mice treated IV with the CAR or mock transduced T cells was monitored.
**FIGS. 13A-13E** show inducible CAR constructs. FIG. 13A shows the shorthand of four inducible CCR4 CAR constructs. FIG. 13B shows the plasmid map of inducible CAR1 (CCR4 EQ with CD19t). FIG. 13C shows the plasmid map of inducible CAR2 (CCR4 CH3 with CD19t). FIG. 13D shows the plasmid map of inducible CAR3 (CCR4 EQ). FIG. 13E shows the plasmid map of inducible CAR4 (CCR4 CH3; also called CCR4 ΔCH2).

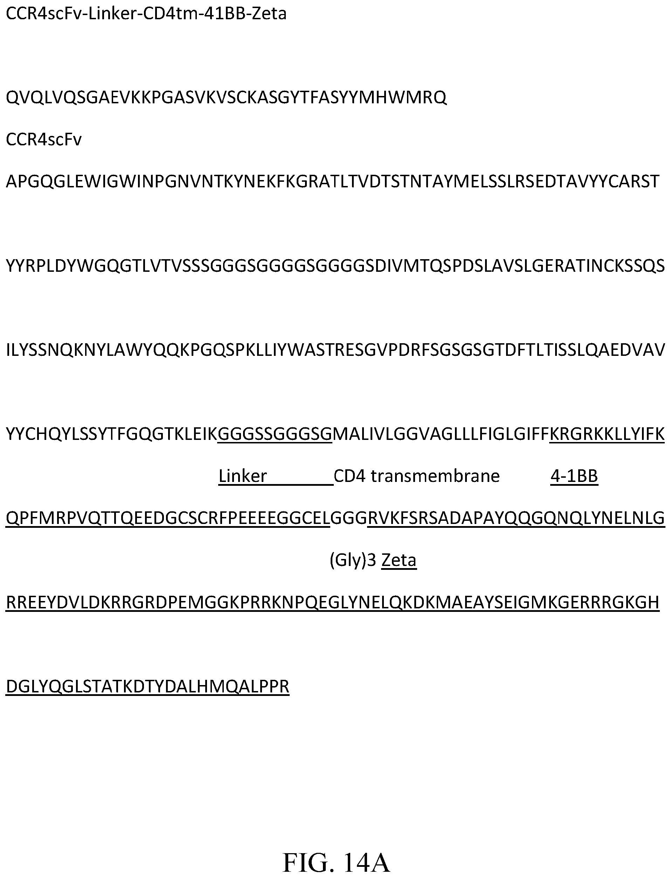

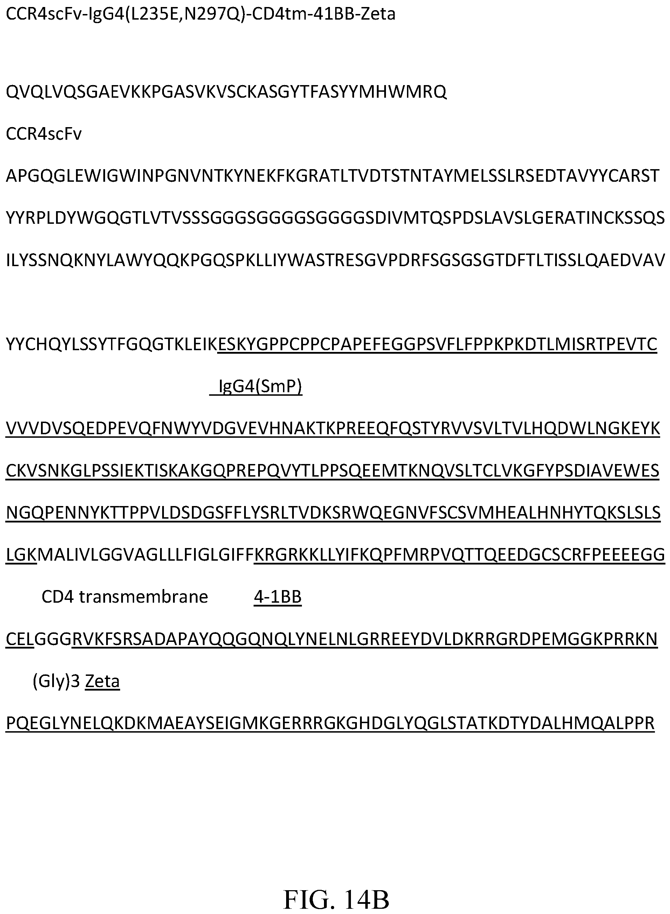

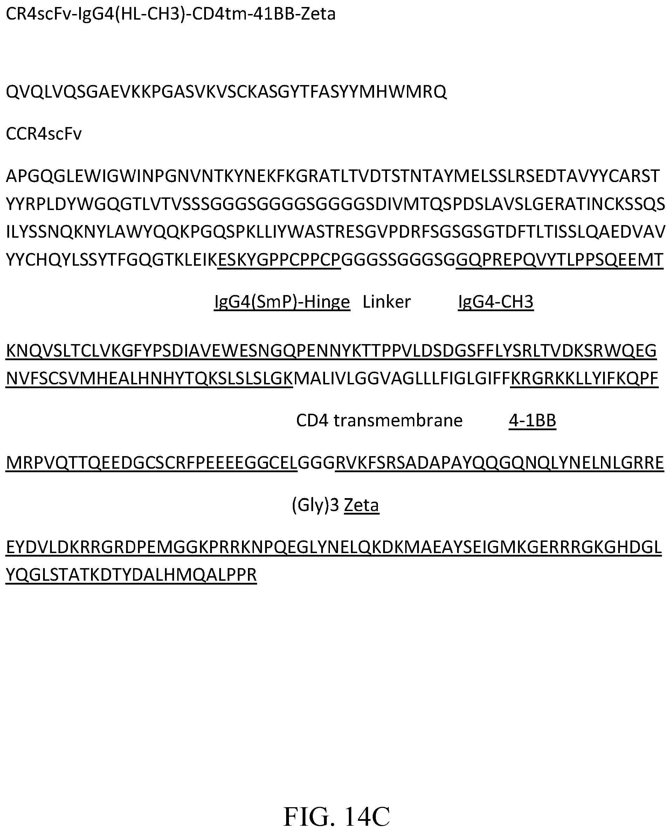

**FIGS 14A-14C** show the annotated amino acid sequences (without the signal sequence) of CAR1 (CCR4 L CAR) (A; SEQ ID NO: 38) CAR2 (CCR4 EQ CAR) (B; SEQ ID NO: 39), and CAR3 (CCR4 ΔCH2 CAR; also called CCR4 CH3 CAR) (C; SEQ ID NO: 40).

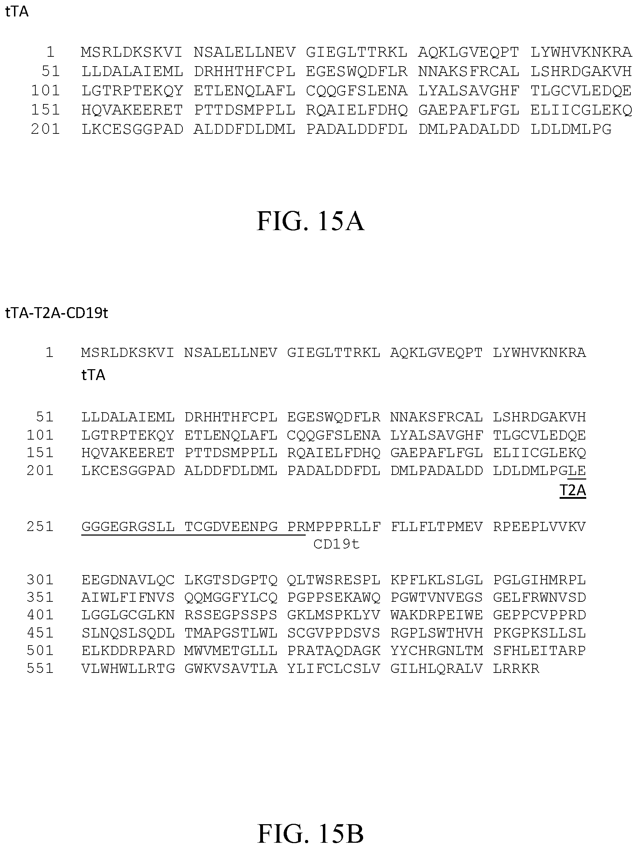

**FIGS. 15A-15B** show the annotated amino acid sequences of tTA (A; SEQ ID NO: 41) and T2A-CD19t (B; SEQ ID NO: 42) as used in the plasmid maps.

### DETAILED DESCRIPTION

In this disclosure the generation and anti-tumor efficacy of CAR with a humanized antihuman CCR4 scFv antigen-binding domain and a 4-1BB intracellular co- stimulatory signaling domain are described. The CCR4 CAR T cells exhibited potent antigen-dependent cytotoxicity against multiple CCR4-expressing human T cell cancer lines. Intravenous *in vivo* delivery of CCR4 CAR T cells in human leukemia/lymphoma murine tumor models conferred elimination of antigen-positive disease and extension of overall survival.

The present disclosure also provides methods for treating subjects with a T-cell cancer, including a non-Hodgkin lymphoma, a peripheral T-cell lymphoma (PTCL), an anaplastic large cell lymphoma, a lympoblastic lymphoma, precursor T-lymphoblastic lymphoma, an angioimmunoblastic T-cell lymphoma, Cutaneous T-Cell Lymphoma (CTCL), mycosis fungoides (MF), Sézary syndrome (SS), and the like. T cell lymphomas encompass a variety of conditions including without limitation: (a) lymphoblastic lymphomas in which the malignancy occurs in primitive lymphoid progenitors from the thymus; (b) mature or peripheral T cell neoplasms, including T cell prolymphocytic leukemia, T- cell granular lymphocytic leukemia, NK-cell leukemia, cutaneous T cell lymphoma (Mycosis fungoides and Sezary syndrome), anaplastic large cell lymphoma, T cell type, enteropathy- type T cell lymphoma, Adult T-cell leukemia/lymphoma including those associated with HTLV-1, and angioimmunoblastic T cell lymphoma, and subcutaneous panniculitic T cell lymphoma; and (c) peripheral T cell lymphomas that initially involve a lymph node paracortex and never grow into a true follicular pattern.

### EXAMPLES

The invention is further described in the following examples, which do not limit the scope of the invention described in the claims.

### Materials and Methods

The following materials and methods were used in the Examples set forth herein.

### Cell Lines

The CEM (adult T cell leukemia line), MT-1 (adult T cell leukemia line), and LCL (generated from PBMC; Engraftment of human central memory-derived effector CD8+ T cells in immunodeficient mice. Xiuli Wang, et al. (2011) Blood) cell lines were cultured in RPMI-1640 (Lonza) containing 10% fetal bovine serum (FBS, Hyclone) (complete RPMI). The 293T cell lines were cultured in Dulbecco's Modified Eagles Medium (DMEM, Life Technologies) containing 10% FBS, 1X AA, 25 mM HEPES (Irvine Scientific), and 2 mM L-Glutamine (Fisher Scientific) (complete DMEM). All cells were cultured at 37°C with 5% CO₂. HUT78 cells were cultured in IMDM (Iscove's Modified Dulbecco's Medium; Fisher Scientific) with 20%FBS.

### DNA Constructs and Lentivirus Production

Tumor cells were engineered to express enhanced green fluorescent protein and firefly luciferase (eGFP/ffluc) by transduction with epHIV7 lentivirus carrying the eGFP/ffluc fusion under the control of the EF1α promoter as described previously (Lenalidomide Enhances the Function of CS1 Chimeric Antigen Receptor-Redirected T Cells Against Multiple Myeloma (Wang et al). Clinical Cancer Research 2018). The humanized scFv sequence used in the CAR construct was obtained from a monoclonal antibody clone h1567 that targets CCR4 (Chang D-H, Sui J, Geng S, Muvaffak A, Bai M, Fuhlbrigge RC, et al. Humanization of an anti-CCR4 antibody that kills Cutaneous T-Cell Lymphoma cells and abrogates suppression by T-regulatory cells. Mol. Cancer Ther. 2012; 11:2451-61).

Lentivirus was generated using a modified polyethylenimine (PEI) mediated transfection method (Optimization of lentiviral vector production using polyethylenimine-mediated transfection. Yong Tang, et al. Oncology Letters. 2015). Briefly, 293T cells were transfected with packaging plasmid and CAR lentiviral backbone plasmid using a modified PEI method. Viral supernatants were collected after 3 to 4 days. Supernatants were concentrated via highspeed centrifugation and lentiviral pellets were resuspended in phosphate-buffered saline (PBS)-lactose solution (4g lactose per 100 mL PBS), aliquoted and stored at -80°C. Lentiviral titers were quantified using jurkat cells based on EGFRt expression.

### T Cell Isolation, Lentiviral Transduction, and Ex Vivo Expansion

Leukapheresis products were obtained from consented research participants (healthy donors) under protocols approved by the City of Hope Internal Review Board (IRB). On the day of leukapheresis, peripheral blood mononuclear cells (PBMC) were isolated by density gradient centrifugation over Ficoll-Paque (GE Healthcare) followed by multiple washes in PBS/EDTA (Miltenyi Biotec). Cells were rested overnight at room temperature (RT) on a rotator, and subsequently washed and resuspended in X-VIVO T cell medium (Lonza) containing 10% FBS (complete X-VIVO). Up to 5.0 x 10⁹ PBMC were incubated with anti-CD14 and anti-CD25 microbeads (Miltenyi Biotec) for 30 min at RT and magnetically depleted using the CliniMACS^{®} system (Miltenyi Biotec) according to the manufacturer's protocol and these were termed depleted PBMCs (dPBMC). dPBMC were frozen in CryoStor^{®} CS5 (StemCell Technologies) until further processing.

T cell activation and transduction was performed as described previously (Co-stimulatory signaling determines tumor antigen sensitivity and persistence of CAR T cells targeting PSCA+ metastatic prostate cancer. Priceman Saul J, et al. 2018. Oncoimmunology). Briefly, freshly thawed dPBMC were washed once and cultured in complete X-VIVO containing 100 U/mL recombinant human IL-2 (rhIL-2, Novartis Oncology) and 0.5 ng/mL recombinant human IL-15 (rhIL-15, CellGenix). For CAR lentiviral transduction, T cells were cultured with CD3/CD28 Dynabeads^{®} (Life Technologies), protamine sulfate (APP Pharmaceuticals), cytokine mixture (as stated above) and desired lentivirus at a multiplicity or infection (MOI) of 1-3 the day following bead stimulation. Cells were then cultured in and replenished with fresh complete X-VIVO containing cytokines every 2-3 days. After 7 days, beads were magnetically removed, and cells were further expanded in complete X-VIVO containing cytokines to achieve desired cell yield. Following further expansion, cells were frozen in CryoStor^{®} CS5 prior to in vitro functional assays and in vivo tumor models. Purity and phenotype of CAR T cells were verified by flow cytometry.

### Flow Cytometry

For flow cytometric analysis, cells were resuspended in FACS buffer (Hank's balanced salt solution without Ca2+, Mg2+, or phenol red (HBSS-/-, Life Technologies) containing 2% FBS and 1x AA). Cells were incubated with primary antibodies for 30 minutes at 4°C in the dark. For secondary staining, cells were washed twice prior to 30 min incubation at 4°C in the dark with either Brilliant Violet 510 (BV510), fluorescein isothiocyanate (FITC), phycoerythrin (PE), peridinin chlorophyll protein complex (PerCP), PerCP-Cy5.5, PE-Cy7, allophycocyanin (APC), or APC-Cy7 (or APC-eFluor780)-conjugated antibodies. Antibodies against CD3 (BD Biosciences, Clone: SK7), CD4 (BD Biosciences, Clone: SK3), CD8 (BD Biosciences, Clone: SK1), CD14 (BD Biosciences, Clone: MΦP9), CD19 (BD Biosciences, Clone: SJ25C1), CD25 (BD Biosciences, Clone: 2A3), mouse CD45 (BioLegend, Clone: 30-F11), CD45 (BD Biosciences, Clone: 2D1), CD69 (BD Biosciences, Clone: L78), CD137 (BD Biosciences, Clone: 4B4-1), MUC1 (BioLegend, Clone 16A), biotinylated Protein-L (GenScript USA), CCR4 (Clone, L291H4), and streptavidin (BD Biosciences) were used. Cell viability was determined using 4', 6-diamidino-2-phenylindole (DAPI, Sigma). Flow cytometry was performed on a MACSQuant Analyzer 10 (Miltenyi Biotec), and the data was analyzed with FlowJo software (v10, TreeStar).

### In Vitro Tumor Killing and Rechallenge Assays

For tumor killing assays, CAR T cells and tumor targets were co-cultured at indicated effector:tumor (E:T) ratios in complete X-VIVO in the absence of exogenous cytokines in 96-well plates for 24 to 72 h and analyzed by flow cytometry as described above. Tumor killing by CAR T cells was calculated by comparing GFP positive tumor cell counts relative to that observed when targets were co-cultured with Mock (untransduced) T cells. For rechallenge assays, 24-72 hours after completion of the killing assay, CAR T cells and tumor targets were again co-cultured at indicated effector:tumor (E:T) ratios in complete X-VIVO in the absence of exogenous cytokines in 96-well plates for 24 to 72 h and analyzed by flow cytometry as described above.

### In Vivo Tumor Studies

All animal experiments were performed under protocols approved by the City of Hope Institutional Animal Care and Use Committee. For *in vivo* tumor studies, CEM cells (3.0 x 10⁶) were prepared in a final volume of 150µl HBSS-/- and engrafted in 6 to 8 week old female or male NSG mice by injection. In some embodiments, engraftment comprises subcutantous (s.c.) injection or intravenous (i.v.) injection. Tumor growth was monitored at least once a week via biophotonic imaging (Xenogen, LagoX) and flux signals were analyzed with Living Image software (Xenogen). For imaging, mice were i.p. injected with 150 µL D-luciferin potassium salt (Perkin Elmer) suspended in PBS at 4.29 mg/mouse. Once flux signals reached desired levels, day 4 or 5 ,T cells were prepared in 1X PBS, and mice were treated with 150 µL intravenous (i.v.) injection of 3.0 x 10⁶ Mock or CCR4 CAR2 T cells. In the CEM tumor model, the impact of treatment with i.v. CCR4 EQ CAR T cells was examined starting at day 4. Humane endpoints were used in determining survival. Mice were euthanized upon signs of distress such as labored or difficulty breathing, apparent weight loss, impaired mobility, or evidence of being moribund. At pre-determined time points or at moribund status, mice were euthanized and tissues were harvested and processed for flow cytometry and/or immunohistochemistry as described below.

Peripheral blood was collected from isoflurane-anesthetized mice by retro-orbital (RO) bleed through heparinized capillary tubes (Chase Scientific) into polystyrene tubes containing a heparin/PBS solution (1000 units/mL, Sagent Pharmaceuticals). Volume of each RO blood draw (approximately 120 µL/mouse) was recorded for cell quantification per µL blood. Red blood cells (RBCs) were lysed with 1X Red Cell Lysis Buffer (Sigma) according to the manufacturer's protocol and then washed, stained, and analyzed by flow cytometry as described above.

### Example 1: Construction of CCR4 CAR T cells containing differing linkers

The studies described below show that CCR4 CAR can be stably expressed on primary T cells.

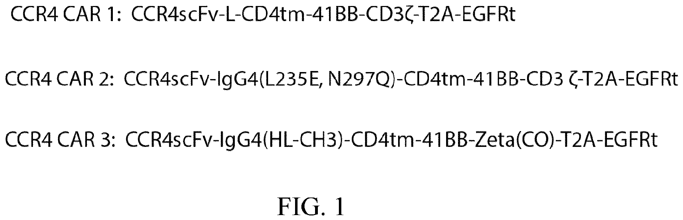

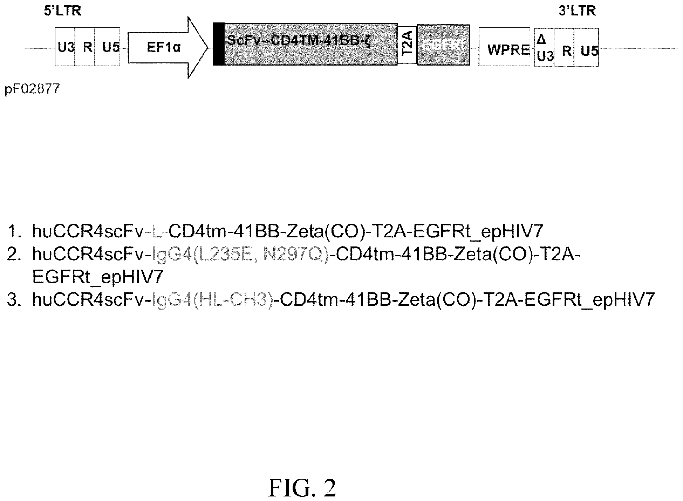

Three CCR4 targeting CAR constructs were designed **(****FIGS. 1** **and** **2****).** All three of the constructs expressed the same codon optimized, humanized CCR4 single chain variable fragment. The CAR constructs also included a CD4 transmembrane domain (TM), a 41BB costimulatory domain, a CD3 zeta domain. The CARs were co-expressed with truncated EGFR, which serve as a marker for the successful transduction of the cells with the CAR construct. The three CCR4 CAR constructs differ in their linker **(****FIG. 2****).** Without being bound by theory, differing lengths in the extracellular portion of the construct may provide differences in the CARs ability to bind the antigen and transmit activation signals after antigen binding. These differences could also result differential killing of CCR4 expressing tumor cells **(****FIG. 1****).**

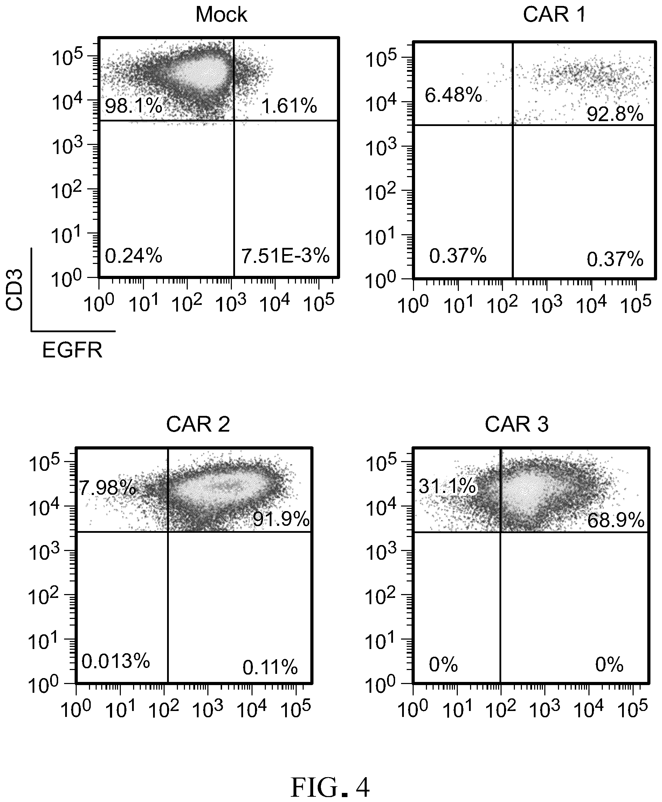

CCR4 CAR lentivirus was used to transduce human healthy donor-derived peripheral blood mononuclear cells depleted of CD14+ and CD25+ cells (dPBMC), as previously described (Priceman SJ, Gerdts EA, Tilakawardane D, Kennewick KT, Murad JP, Park AK, Jeang B, Yamaguchi Y, Yang X, Urak R, Weng L, Chang WC, Wright S, Pal S, Reiter RE, Wu AM, Brown CE, Forman SJ. Co-stimulatory signaling determines tumor antigen sensitivity and persistence of CAR T cells targeting PSCA+ metastatic prostate cancer. Oncoimmunology. 2018; 7(2):e1380764) as well as other cells types such as enriched T-cells (EasySep Human T cell isolation Kit. StemCell Technologies). Using EGFRt as a tracking marker, flow cytometry was used to show CAR expression as described above. All three CCR4 CAR constructs were stably expressed in T cells **(****FIG. 4****).** Seven days after dPBMC cells were transduced, cells were stained with anti-CD3 to mark T cells and anti-EGFR to mark successful incorporation of the CCR4 CAR construct. CCR4 L CAR, CCR4 EQ CAR, and CCR4 ΔCH2 CAR were all stably expressed at 7 days post-transduction **(****FIG. 4****)** and expression of EGFR was shown to be stable up to 28 days after the start of transduction (data not shown).

### Example 2: CCR4 expression in T cell populations

The studies described below examined CCR4 CAR T cell expansion and activity in different T cell subpopulations, such as PBMC (CD14-, CD25-), and pan-T cells.

The starting T cell population used to generate CAR T cells may influence the potency with which the CAR T cells can eliminate its target cells. There may also be differences in the proliferation of the cells during the 14 day manufacturing process.

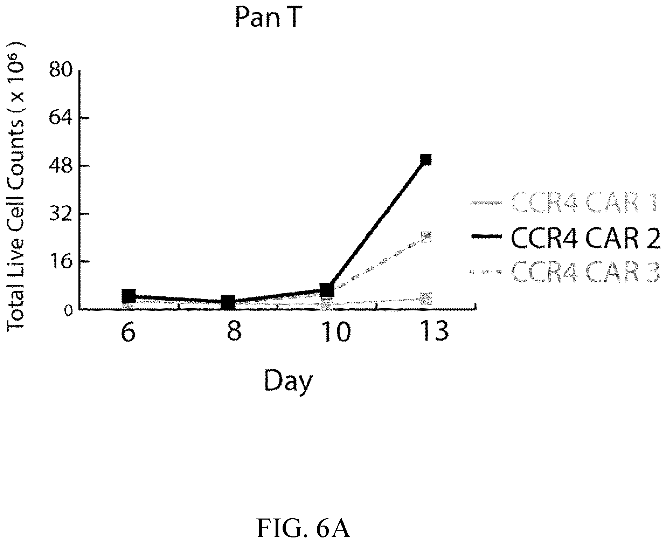

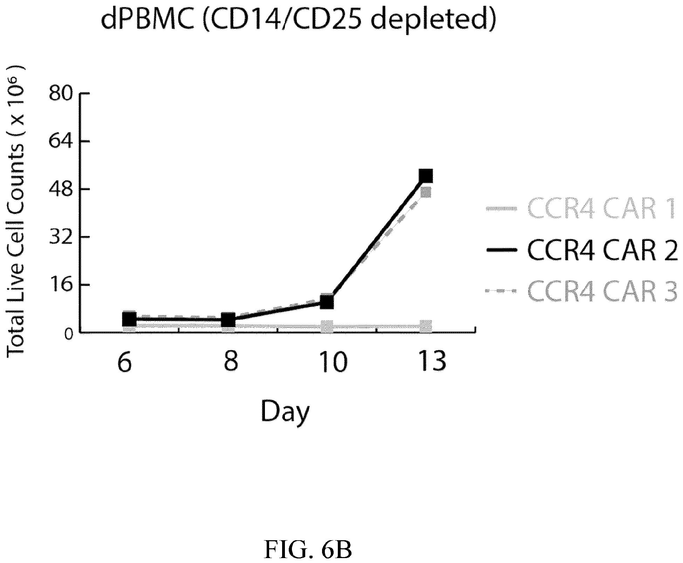

We transduced different populations of T cells from the same healthy donor with the 3 different CCR4 CAR constructs **(****FIG. 1****)** and tracked the live cells counts of the cells over 13 days. We found that CCR4 EQ CAR and CCR4 ΔCH2 CAR proliferated the best overall **(****FIGS. 6A-6B****).** We have observed that CCR4 L CAR has poorer proliferation in several independent studies with CCR4 L CAR cells generated from different healthy donors in a variety of starting T cell populations (data not shown).

### Example 3: Validation that CCR4 CAR T cells selectively target CCR4-positive cells in vitro

To determine if CCR4 CAR T cells demonstrate selective activity against CCR4-positive cancer cells, the CCR4 CAR T cells were grown in presence of either CCR4-positive or CCR4-negative cancer cells and the percentage of cancerous cells killed was quantified.

To assess antigen-dependent activity of our CCR4 CAR T cells, co-cultured assays with CCR4-positive and -negative tumor targets were conducted at an E:T ratio between 1:2 and 1:10 to determine their killing potential. The CCR4-positive T cell tumor lines used were MT-1 and CEM. The CCR4-negative cell line used was LCL, a B cell tumor cell line. We co-cultured CCR4 EQ CAR T cells and tumor cells at a ratio of 1 T cell for every 10 tumor cells (1:10 E:T). Using flow cytometry we then tested for the killing of tumor cells (% specific lysis) after 48 hours.

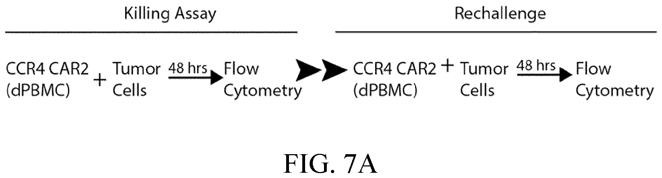

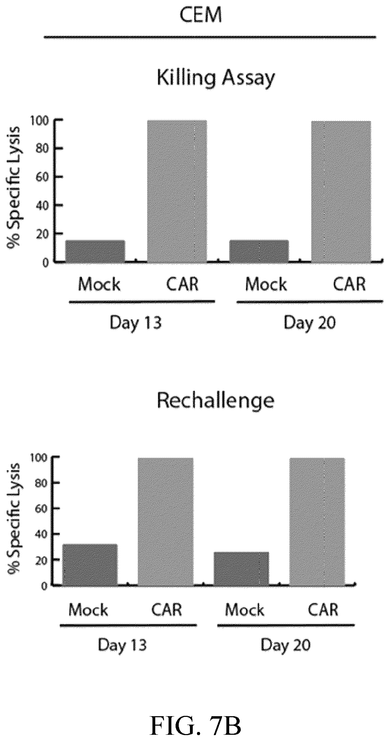

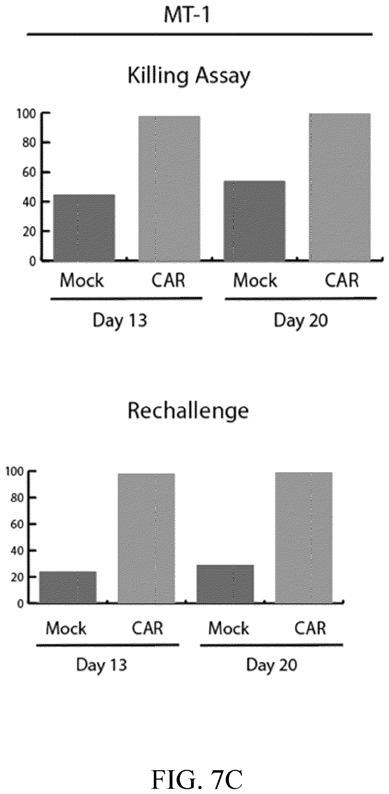

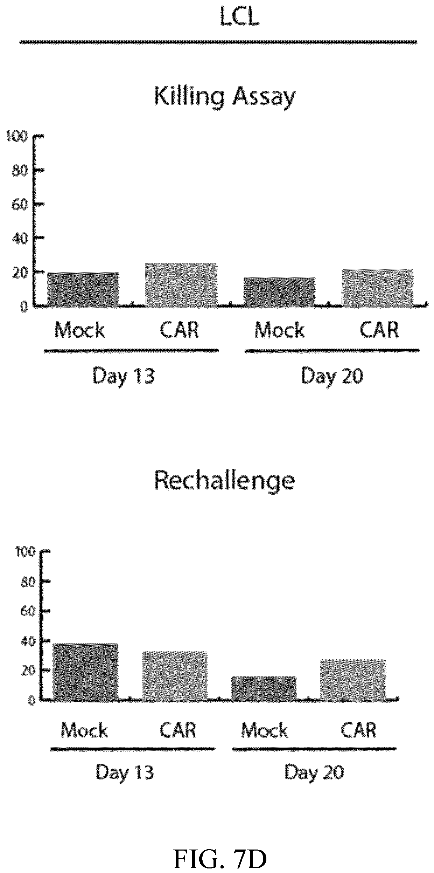

After 48 hours, antigen-specific T cell-mediated killing activity was evident with CCR4 EQ CAR T cells relative to Mock T cells in the CEM tumor line **(****FIG. 7B****,** top) and the MT-1 tumor cell line **(****FIG. 7C****,** top). CCR4 EQ CAR2 T cells had sustained killing at or near 100% lysis in both the CEM tumor line **(****FIG. 7B****,** top) and the MT-1 tumor cell line (**FIG. 7C****,** top). These results at an E:T of 1:10 demonstrate the potent killing ability of CCR4 EQ CAR T cells. CCR4 EQ CAR T cells showed minimal killing of CCR4-negative LCL cells **(****FIG. 7D****,** top). CCR4 L CAR T cells and CCR4 ΔCH2 CAR T cells also killed CCR4+ tumor cells (data not shown).

Additionally, to test if CCR4 EQ CAR T cells can continue to kill tumor cells if rechallenged, we co-cultured CAR T cells and tumor cells at a ratio of 1:2 (1 T cell:2 tumor cells) for 48 hrs. After the 48 hours, we added additional tumor cells to the co-culture wells to an E:T of 1:2, and after an additional 48 hours, used flow cytometry to determine the % specific lysis of the tumor cells. Surprisingly, we found that the CCR4 EQ CAR T cells were able to kill CCR4+ tumor lines even when rechallenged **(****FIGS. 7B-7C****,** bottom). Both CCR4 L CAR T cells and CCR4 ΔCH2 CAR T cells also killed CCR4+ tumor cells when rechallenged (data not shown).

### Example 4: Establishment of in vivo malignant T cell mouse models

To evaluate the therapeutic potential of the CCCR4 CAR T cells *in vivo,* three mouse models were established using a variety of injection routes.

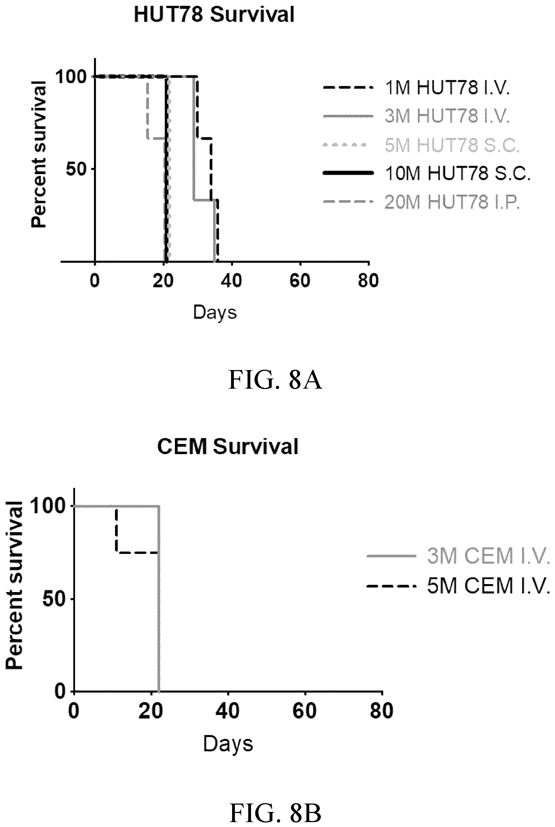

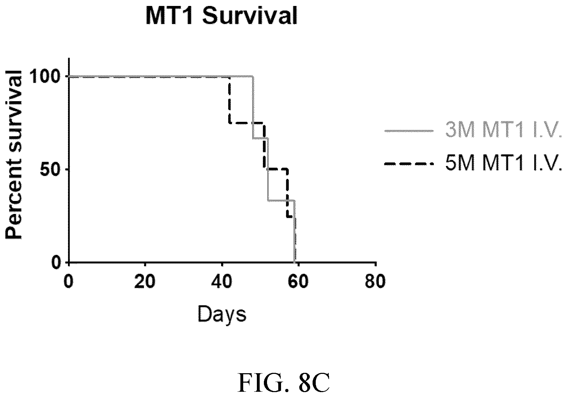

Humane endpoints were used in determining survival curves of NSG mice engrafted with CCR4 expressing malignant T cell lines. Mice were euthanized upon signs of distress such as a distended belly due to ascites, labored or difficulty breathing, apparent weight loss, impaired mobility, or evidence of being moribund. Mice were engrafted cells delivered systemically or locally with HUT78, CEM, and MT-1 via i.p., s.c., and i.v. injection **(****FIG. 8****).**

### Example 5: Validation that CCR4 CAR T cells delivered in vivo in a mouse model exhibit potent anti-tumor activity and confer extended lifespan to the mice

To evaluate *in vivo* efficacy of CCR4 CAR T cells to selectively target CCR4-positive cells in the CEM model, CCR4 CAR T cells were delivered and tumor size and survival was evaluated over time.

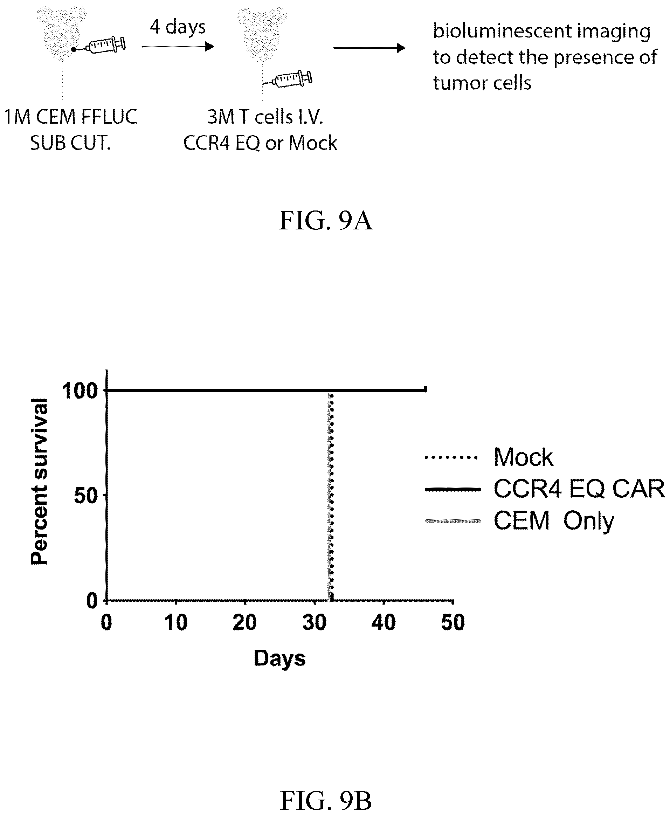

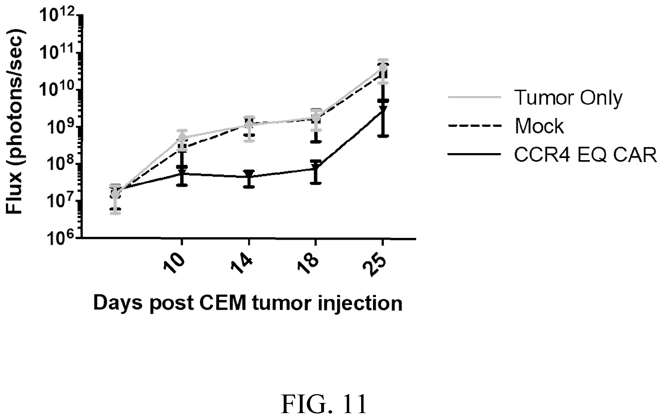

CEM cells were lentivirally transduced to express firefly luciferase (ffluc) to allow for tracking of tumor growth via non-invasive optical imaging. At 4 days post tumor s.c. injection, mice were treated with Mock or CCR4 EQ CAR2 T cells (3.0 x 10⁶) by systemic intravenous (i.v.) delivery **(****FIG. 9A****).** Rapid anti-tumor effects were observed in mice treated with CCR4 EQ CAR T cells via i.v. delivery, reaching a maximal anti-tumor response 1-2 weeks following treatment. Anti-tumor responses in mice were durable for 3-4 weeks, but ultimately tumor recurrences were observed in mice. Delivery of CCR4 EQ CAR T cells significantly extended survival of mice **(****FIG. 9B** and **FIG. 11****).**

### Example 6: Validation that CCR4 CAR T cells delivered in vivo in a mouse model exhibit potent anti-tumor activity and confer extended lifespan to the mice

To evaluate *in vivo* efficacy of CCR4 CAR T cells in a disseminated model, CCR4 CAR T cells were delivered to the HUT78 mouse model, and tumor size and survival was evaluated over time.

HUT78 were cultured in IMDM (Iscove's Modified Dulbecco's Medium; Fisher Scientific) with 20%FBS. For the HUT78 *in vivo* model, CD4 and CD8 enriched cells from PBMC via incubation of PBMC with anti-CD4 and anti-CD8 microbeads (Miltenyi Biotech). Another T cell population used were T cells generated by negative selection of PBMC. Human T cell isolation kit from StemCell was also used.

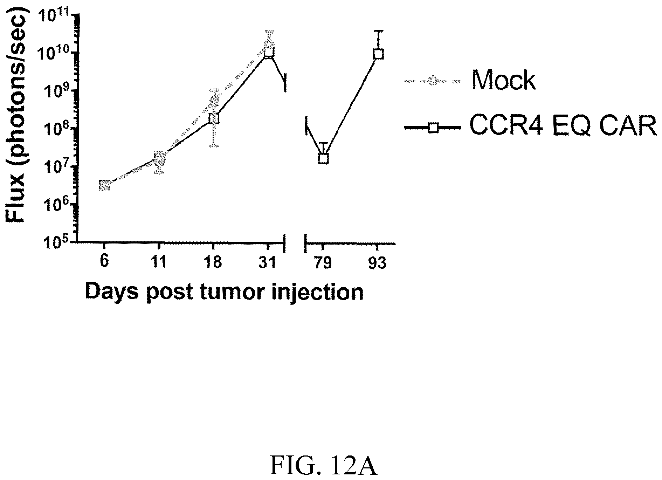

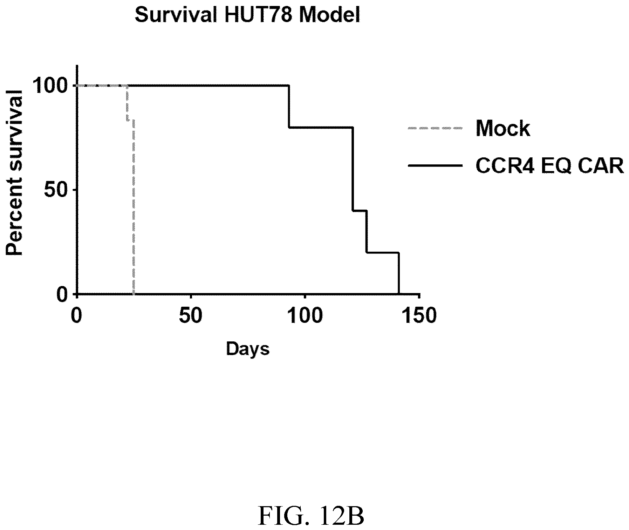

For *in vivo* tumor studies, HUT78 cells (1.0 x 10⁶) were prepared in a final volume of 150 µl HBSS-/- and engrafted in 6 to 8 week old female or male NSG mice by injection. HUT78 Animal model is a disseminated model. In some embodiments, engraftment comprises subcutantous (s.c.) injection or intravenous (i.v.) injection. HUT78 cells were lentivirally transduced to express firefly luciferase (ffluc) to allow for tracking of tumor growth via non-invasive optical imaging. At 10 days post tumor i.v. injection, mice were treated with Mock or CCR4 EQ CAR2 T cells (3.0 x 10⁶) by systemic intravenous (i.v.) **(****FIG. 12A****).** Anti-tumor effects were observed in mice treated with CCR4 EQ CAR T cells following treatment. Delivery of CCR4 EQ CAR T cells significantly extended survival of mice **(****FIG. 12B****).**

### Example 7: Inducible CCR4 CAR T cells

In some circumstances it is desirable to control expression of a CCR4 CAR. For example, after a vector is introduced into population of T cells, the cells are expanded to prepare a sufficient number of cells to use therapeutically. During this expansion stage, it can be desirable to reduce or nearly eliminate expression of the CCR4 CAR, for example, to reduce any fratricide. A system that uses Tet-Off control can be useful (Das et al. 2016 Current Gene Therapy 16:156). Thus, an expression vector for expression of a CCR4 CAR can express the CCR4 CAR under the control of an inducible promoter that includes several copies of the tet operator and minimal promoter. The vector can also encode a fusion protein comprising the transcription activation of domain of VP16 fused to TetR. In the presence of tetracycline or doxycycline, expression of the CCR4 CAR is repressed. When tetracycline or doxycycline is not present, the CCR4 is expressed. Thus, T cells harboring a nucleic acid encoding CCR4 CAR can be expanded under conditions in which CCR4 CAR expression is repressed. When a desired number of cells is obtained, tetracycline or doxycycline is withdrawn to induce expression.

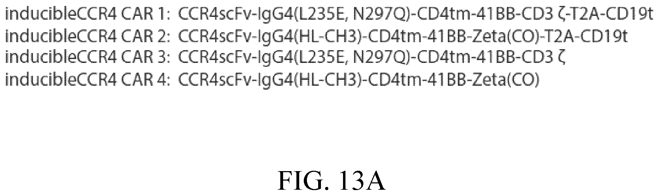

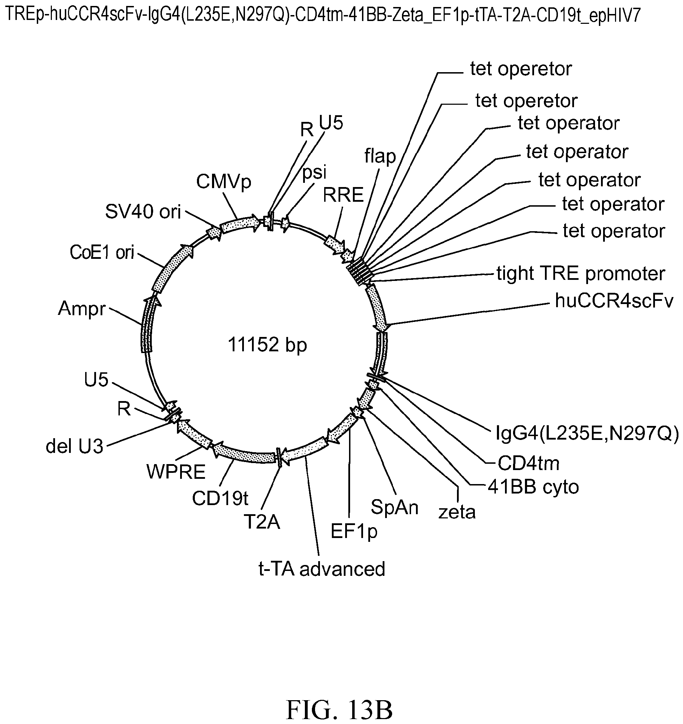

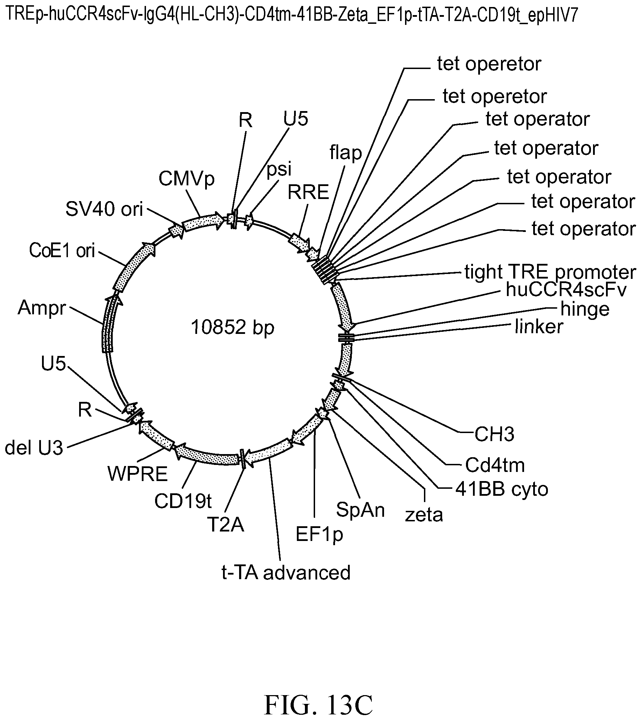

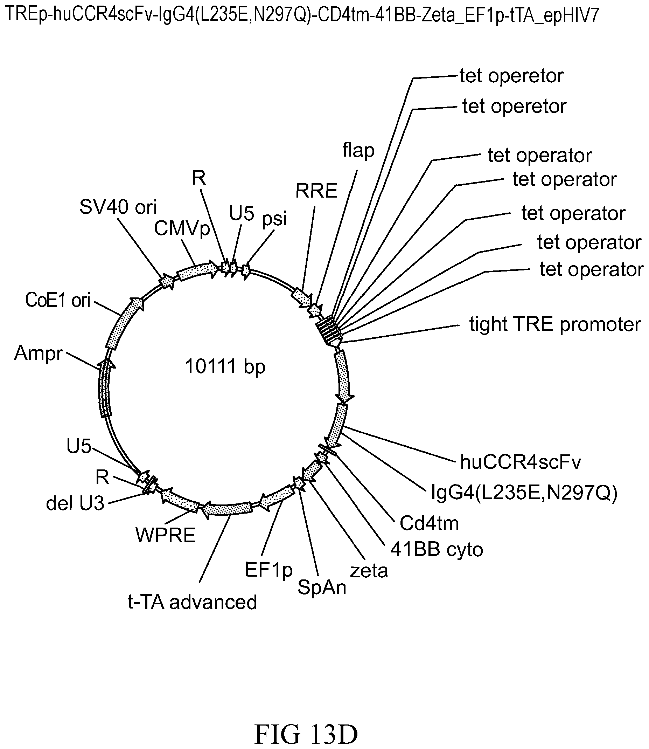

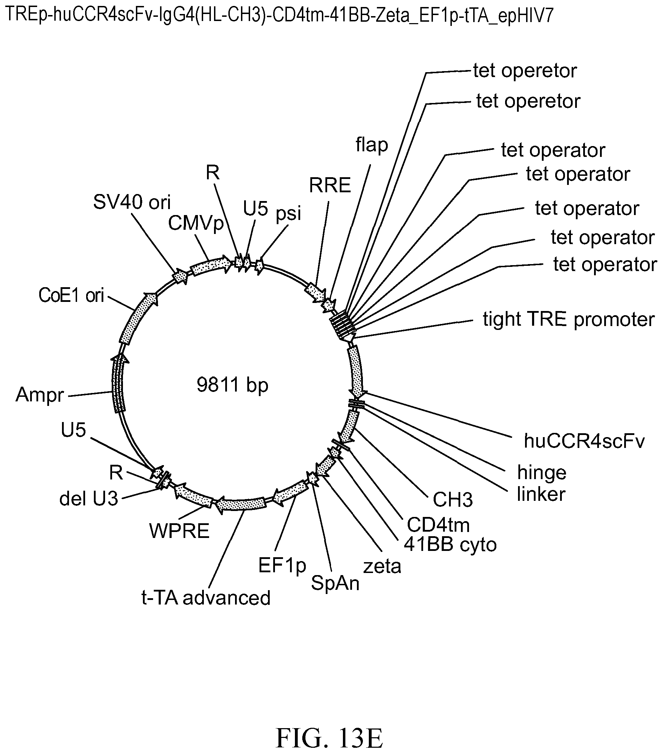

**FIG. 13A** depicts schematic diagrams of four inducible CCR4 CAR constructs. **FIG. 13B** shows the plasmid map of inducible CAR1 (CCR4 EQ with CD19t). **FIG. 13C** shows the plasmid map of inducible CAR2 (CCR4 CH3 with CD19t). **FIG. 13D** shows the plasmid map of inducible CAR3 (CCR4 EQ). **FIG. 13E** shows the plasmid map of inducible CAR4 (CCR4 CH3).

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

All references are herein incorporated in their entirety for any and all purposes.

### EMBODIMENTS

Although the present invention is defined in the claims, it should be understood that the present invention can also (alternatively) be defined in accordance with the following embodiments:
1. A nucleic acid molecule comprising a nucleotide sequence encoding a chimeric antigen receptor (CAR) or a polypeptide, wherein the chimeric antigen receptor or polypeptide comprises: an scFv targeting CCR4, a spacer, a transmembrane domain, a 4-1BB co-stimulatory domain, and a CD3 ζ signaling domain, wherein the spacer comprises SEQ ID NO:2, SEQ ID NO:9, SEQ ID NO: 10, or SEQ ID NO: 12, or a variant thereof having 1-5 amino acid modifications.
2. The nucleic acid molecule of embodiment 1, wherein the transmembrane domain is selected from: a CD4 transmembrane domain or variant thereof having 1-5 amino acid modifications, a CD8 transmembrane domain or variant thereof having 1-5 amino acid modifications, a CD28 transmembrane domain or a variant thereof having 1-5 amino acid modifications.
3. The nucleic acid molecule of embodiment 1, wherein the scFv comprises the amino acid sequence of SEQ ID NO:32 and the amino acid sequence of SEQ ID NO: 33 or the amino acid sequence of SEQ ID NO:34 and the amino acid sequence of SEQ ID NO: 35 or the amino acid sequence of SEQ ID NO:36 and the amino acid sequence of SEQ ID NO: 37.
4. The nucleic acid molecule of embodiment 1, wherein the transmembrane domain is a CD4 transmembrane domain or variant thereof having 1-5 amino acid modifications.
5. The nucleic acid molecule of embodiment 1, wherein the transmembrane domain is a CD4 transmembrane domain.
6. The nucleic acid molecule of embodiment 1, wherein the chimeric antigen receptor or polypeptide comprises a transmembrane domain selected from: a CD4 transmembrane domain or variant thereof having 1-2 amino acid modifications, a CD8 transmembrane domain or variant thereof having 1-2 amino acid modifications, a CD28 transmembrane domain or a variant thereof having 1-2 amino acid modifications,
7. The nucleic acid molecule of embodiment 1, wherein the 4-1BB costimulatory domain comprises the amino acid sequence of SEQ ID NO: 24 or a variant thereof having 1-5 amino acid modifications.
8. The nucleic acid molecule of embodiment 1, wherein the CD3ζ signaling domain comprises the amino acid sequence of SEQ ID NO:21.
9. The nucleic acid molecule of embodiment 1, wherein a linker of 3 to 15 amino acids is located between the 4-1BB costimulatory domain and the CD3 ζ signaling domain or variant thereof.
10. The nucleic acid molecule of embodiment 1, wherein the CAR or the polypeptide comprises the amino acid sequence of SEQ ID NO: 29, 30, 31, 38, 39, or 40 or a variant thereof having 1-5 amino acid modifications.
11. The nucleic acid molecule of embodiment 1, wherein the scFv comprises the amino acid sequence of any one of SEQ ID NO:1, 40, 43, 44, or 45.
12. An expression vector comprising the nucleic acid molecule of embodiment 1.
13. The expression vector of embodiment 12, wherein the vector is a viral vector.
14. The expression vector of embodiment 12, wherein expression of the CCR4 CAR is under the control of an inducible promoter.
15. The expression vector of embodiment 14, wherein expression of the CCR4 CAR is under the control of a Tet Off system.
16. A population of human T cells transduced by a vector comprising the nucleic acid molecule of embodiment 1.
17. The population of human T cells of embodiment 14, wherein the population of human T cells comprise central memory T cells, naive memory T cells, CD4+ cells and CD8+ cells enriched from PBMC cells, T cells isolated via negative depletion, or PBMC substantially depleted for CD25+ cells and CD14+ cells.
18. A method of treating T cell lymphoma in a patient comprising administering a population of autologous or allogeneic human T cells transduced by a vector comprising the nucleic acid molecule of embodiment 1, wherein the T cell lymphoma comprises cells expressing CCR4.
19. The method of embodiment 18, wherein the population of human T cells expressing the chimeric antigen receptor or the polypeptide is administered locally or systemically.
20. The method of embodiment 18, wherein the CCR4-expressing cells are cancerous T cells or T-regulatory cells.
21. The method of embodiment 18, wherein the population of human T cells expressing the chimeric antigen receptor or the polypeptide is administered by single or repeat dosing.
22. A method of preparing CCR4 CAR T cells comprising:
   providing a population of autologous or allogeneic human T cells and
   transducing the T cells by a vector comprising the nucleic acid molecule of embodiment 1,
   wherein the T cells comprise PBMC cells.
23. The method of embodiment 22, wherein the depleted PBMC cells are at least about 80%, 85%, 90%, 95%, 96%, 97%, 98%, 99%, or 100% CD14 negative and at least about 80%, 85%, 90%, 95%, 96%, 97%, 98%, 99%, or 100% CD25 negative.
24. The method of embodiment 21, wherein the PBMC cells comprise CD4+ T cells or CD8+ T cells or both
25. The population of human T cells of embodiment 14, wherein the population of human T cells comprise PBMC cells are at least about 80%, 85%, 90%, 95%, 96%, 97%, 98%, 99%, or 100% CD14 negative and at least about 80%, 85%, 90%, 95%, 96%, 97%, 98%, 99%, or 100% CD25 negative.
26. A method of preparing T cells expressing a CCR4 CAR, comprising expanding T cells harboring the expression vector of embodiment 14 under conditions in which CCR4 CAR expression is not induced until a desired number of cells is produced and then inducing CCR4 CAR expression.

## Claims

1. A nucleic acid molecule comprising a nucleotide sequence encoding a chimeric antigen receptor (CAR) or a polypeptide, wherein the chimeric antigen receptor or polypeptide comprises: an scFv targeting CCR4, a spacer, a transmembrane domain, a 4-1BB co-stimulatory domain, and a CD3 ζ signaling domain, wherein the spacer comprises SEQ ID NO:9, or a variant thereof having 1-5 amino acid modifications.

2. The nucleic acid molecule of claim 1, wherein the transmembrane domain is selected from: a CD4 transmembrane domain or variant thereof having 1-5 amino acid modifications, a CD8 transmembrane domain or variant thereof having 1-5 amino acid modifications, a CD28 transmembrane domain or a variant thereof having 1-5 amino acid modifications, optionally wherein the transmembrane domain is a CD4 transmembrane domain.

3. The nucleic acid molecule of claim 1, wherein the scFv comprises the amino acid sequence of SEQ ID NO:32 and the amino acid sequence of SEQ ID NO: 33 or the amino acid sequence of SEQ ID NO:34 and the amino acid sequence of SEQ ID NO: 35 or the amino acid sequence of SEQ ID NO:36 and the amino acid sequence of SEQ ID NO: 37, or
wherein the scFv comprises the amino acid sequence of any one of SEQ ID NO: 1, 40, 43, 44, or 45.

4. The nucleic acid molecule of claim 1, wherein the 4-1BB costimulatory domain comprises the amino acid sequence of SEQ ID NO: 24 or a variant thereof having 1-5 amino acid modifications, and/or wherein the CD3ζ signaling domain comprises the amino acid sequence of SEQ ID NO:21, and/or wherein a linker of 3 to 15 amino acids is located between the 4-1BB costimulatory domain and the CD3 ζ signaling domain or variant thereof.

5. The nucleic acid molecule of claim 1, wherein the CAR or the polypeptide comprises the amino acid sequence of SEQ ID NO: 31 or 40 or a variant thereof having 1-5 amino acid modifications.

6. An expression vector comprising the nucleic acid molecule of any one of claims 1-5.

7. The expression vector of claim 6, wherein the vector is a viral vector, and/or wherein expression of the CCR4 CAR is under the control of an inducible promoter, optionally wherein expression of the CCR4 CAR is under the control of a Tet Off system.

8. A population of human T cells transduced by a vector comprising the nucleic acid molecule of any one of claims 1-5.

9. The population of human T cells of claim 8, wherein the population of human T cells comprise central memory T cells, naive memory T cells, CD4+ cells and CD8+ cells enriched from PBMC cells, T cells isolated via negative depletion, or PBMC substantially depleted for CD25+ cells and CD14+ cells, or
optionally wherein the population of human T cells comprise PBMC cells are at least about 80%, 85%, 90%, 95%, 96%, 97%, 98%, 99%, or 100% CD14 negative and at least about 80%, 85%, 90%, 95%, 96%, 97%, 98%, 99%, or 100% CD25 negative.

10. A population of autologous or allogeneic human T cells transduced by a vector comprising the nucleic acid molecule of any one of claims 1-5 for use in a method of treating T cell lymphoma in a patient, the method comprising administering said population to said patient, wherein the T cell lymphoma comprises cells expressing CCR4.

11. The population for use of claim 10, wherein the population of human T cells expressing the chimeric antigen receptor or the polypeptide is administered locally or systemically, and/or
wherein the population of human T cells expressing the chimeric antigen receptor or the polypeptide is administered by single or repeat dosing.

12. The population for use of claim 10, wherein the CCR4-expressing cells are cancerous T cells or T-regulatory cells.

13. A method of preparing CCR4 CAR T cells comprising:
providing a population of autologous or allogeneic human T cells and
transducing the T cells by a vector comprising the nucleic acid molecule of any one of claims 1-5,
wherein the T cells comprise PBMC cells.

14. The method of claim 13, wherein the PBMC cells are at least about 80%, 85%, 90%, 95%, 96%, 97%, 98%, 99%, or 100% CD14 negative and at least about 80%, 85%, 90%, 95%, 96%, 97%, 98%, 99%, or 100% CD25 negative, and/or wherein the PBMC cells comprise CD4+ T cells or CD8+ T cells or both.

15. A method of preparing T cells expressing a CCR4 CAR, comprising expanding T cells harboring the expression vector of claim 6 or claim 7 under conditions in which CCR4 CAR expression is not induced until a desired number of cells is produced and then inducing CCR4 CAR expression.
